# EUROPEAN PATENT APPLICATION

(11) **EP 3 153 523 A2**
(43) Date of publication of application: **12.04.2017**
(21) Application number: 16199824.0
(22) Date of filing: 01.03.2013
(51) Int. Cl.: C07K 14/575, A61K 38/00, A61K 38/22, A61K 45/06

(54) **VARIANTS OF PROTHYMOSIN ALPHA AND METHODS OF USING SAME**

(30) Priority: 02.03.2012 US 201261606193 P
(62) Divisional of application: 13755552.0
(71) Applicant: Icahn School of Medicine at Mount Sinai, New York, NY 10029-6574 (US)
(72) Inventor: MOSOIAN, Arevik, Jackson Heights, NY 11372 (US); TEIXEIRA, Avelino, Montclair, NJ 07042 (US)
(74) Representative: Carpmaels & Ransford LLP

(57) **Abstract**

The present invention relates to novel Prothymosin Alpha (ProTα) variants that are capable of inducing cell-mediated immune responses. These variants lack a nuclear localization signal and the proliferative oncogenic activity previously attributed to ProTα. The variants of the invention are used in methods of treating, for example, viral infections, bacterial infections, fungal infections, cancer, ischemia and myeloproliferative blood disorders. Administration of a ProTα variant to a subject in a therapeutically effective amount treats the infection or disease.

## Description

This application claims priority from U.S. Provisional Application Ser. No. 61/606,193, filed March 2, 2012, the disclosure of which is hereby incorporated by reference in its entirety.

### BACKGROUND OF THE INVENTION

Prothymosin alpha (ProTα) has been isolated from a wide range of human cell types, tissues and bodily fluids and has been reported to have different functions as an intracellular or extracellular protein. The major intracellular activity of ProTα is linked to cell proliferation, as suggested by evidence that it is highly expressed in malignant tumor cells of various cancer types (1-3). Extracellular ProTα, found both in blood and in the growth medium of cultured cells, is reported to be associated with cell-mediated immunity activities (4-6), including anti-viral and anti-bacterial cytokine-like functions (6-11), activation of natural killer (NK) cells, stimulation of tumor-specific cytotoxic T-cells and up-regulation of MHC class II on antigen presenting cells (4, 5, 12-14). The presence of a nuclear localization signal (NLS) directs intracellular ProTα to the nucleus, while ProTα variants with no or defective NLSs are more likely to be released extracellularly in response to viral challenge (15, 16).

Despite ten known ProTα gene-family members in the human genome, only one ProTα-coding gene has been reported to be expressed (this form of ProTα is referred to as the common- or cProTα). ProTα has a wide cellular distribution and is abundantly expressed, similar to the 90-kDa heat shock protein and ribosomal proteins; accordingly, mRNA and protein derived from this gene is easily isolated (17, 18). ProTα has a highly conserved primary structure in all mammalian species, suggesting an essential function for this protein.

Of the ten ProTα gene-family members, eight were considered to be processed pseudogenes, and, until the instant invention, they were considered to be unexpressed. Pseudogenes may arise by two processes termed retrotransposition, which refers to the formation of processed pseudogenes, and duplication. During duplication of a gene, modifications to the DNA sequence (such as mutations, insertions or deletions) can occur at the transcriptional or translational level and render the duplicated gene non-functional such that protein is no longer produced. During formation of a processed pseudogene, an mRNA is reverse transcribed and this cDNA is subsequently reintegrated into the genome. Processed pseudogenes are characterized as having no introns and containing a 3'-poly-A tail as well as directed repeats flanking the pseudogene, however they lack the upstream promoter necessary for expression of a regular gene. These ProTα- pseudogene family members are located on different chromosomes, which carry the necessary gene regulatory elements for transcription and thus these pseudogenes have the potential to be expressed (19, 20). The remaining two ProTα gene-family members are recognized as potential splice-variants of the cProTα gene, and are referred to as isoform A and isoform B. However, no mRNA or protein has previously been identified from any ProTα gene-family members other than the cProTα-coding gene (19, 20).

A recent screen of proteins present in normal donor cervical lavage fluid (CVL) isolated an extracellular form of ProTα that has potent anti-HIV-1 activity (19). Further studies demonstrated that this anti-HIV activity is not induced by the bioactive amino terminal of ProTα spanning 28 amino acids, called Thymosin alpha 1, but instead due to a peptide containing at least the amino acids 50-89 of ProTα (19). Recently, a ProTα variant was identified in CD8⁺ cell-conditioned media as exhibiting potent anti-HIV activity by inducing type I IFN via TLR4 activation, however this IFN-mediated anti-HIV activity was mistakenly attributed to common-cProTα (10).

There is a need for ProTα polypeptides that have the cell-mediated immunity activity of ProTα, without the pro-proliferative activity typically associated with ProTα. The present invention identifies such novel ProTα variants. These variants have interferon-inducing activity that can be used for the treatment of viral infections, bacterial infections and cancer. These variants all lack a functional NLS, which abolishes their nuclear localization, proliferative function, and oncogenic activity.

### SUMMARY OF THE INVENTION

This invention generally relates to isolated prothymosin alpha (ProTα) variants that are capable of inducing cell-mediated immune responses. The ProTα variants lack a nuclear localization signal and proliferative oncogenic activity generally attributed to ProTα. The variants of this invention are used in methods of treating. As an example, the ProTα variants of this invention can be used to treat bacterial, fungal, and viral infections. The ProTα variants of this invention can also be used to treat cancer, ischemia, and myeloproliferative blood disorders.

In certain aspects of the invention, the invention provides an isolated prothymosin alpha polypeptide, wherein the polypeptide comprises an amino acid sequence that is at least about 98% identical to an amino acid sequence of pProTα (SEQ ID NO: 1). In certain aspects of the invention, the invention provides an isolated prothymosin alpha polypeptide, wherein the polypeptide comprises an amino acid sequence that is at least about 95% identical to an amino acid sequence of pProTα (SEQ ID NO: 1). In another aspect, the invention provides an isolated prothymosin alpha polypeptide, wherein the polypeptide comprises an amino acid sequence that is at least about 90% identical to an amino acid sequence of pProTα (SEQ ID NO: 1). In other aspects, the invention provides an isolated prothymosin alpha polypeptide, wherein the polypeptide comprises a functional derivative of pProTα (SEQ ID NO: 1). In yet another aspect, the invention provides an isolated prothymosin alpha polypeptide, wherein the polypeptide comprises a fragment of pProTα (SEQ ID NO: 1).

In certain aspects of the invention, the invention provides an isolated prothymosin alpha polypeptide, wherein the polypeptide comprises an amino acid sequence that is at least about 98% identical to an amino acid sequence of isoA (SEQ ID NO: 2). In certain aspects of the invention, the invention provides an isolated prothymosin alpha polypeptide, wherein the polypeptide comprises an amino acid sequence that is at least about 95% identical to an amino acid sequence of isoA (SEQ ID NO: 2). In another aspect, the invention provides an isolated prothymosin alpha polypeptide, wherein the polypeptide comprises an amino acid sequence that is at least about 90% identical to an amino acid sequence of isoA (SEQ ID NO: 2). In other aspects, the invention provides an isolated prothymosin alpha polypeptide, wherein the polypeptide comprises a functional derivative of isoA (SEQ ID NO: 2). In yet another aspect, the invention provides an isolated prothymosin alpha polypeptide, wherein the polypeptide comprises a fragment of isoA (SEQ ID NO: 2).

In certain aspects of the invention, the invention provides an isolated prothymosin alpha polypeptide, wherein the polypeptide comprises an amino acid sequence that is at least about 98% identical to an amino acid sequence of isoB (SEQ ID NO: 3). In certain aspects of the invention, the invention provides an isolated prothymosin alpha polypeptide, wherein the polypeptide comprises an amino acid sequence that is at least about 95% identical to an amino acid sequence of isoB (SEQ ID NO: 3). In another aspect, the invention provides an isolated prothymosin alpha polypeptide, wherein the polypeptide comprises an amino acid sequence that is at least about 90% identical to an amino acid sequence of isoB (SEQ ID NO: 3). In other aspects, the invention provides an isolated prothymosin alpha polypeptide, wherein the polypeptide comprises a functional derivative of isoB (SEQ ID NO: 3). In yet another aspect, the invention provides an isolated prothymosin alpha polypeptide, wherein the polypeptide comprises a fragment of isoB (SEQ ID NO: 3).

In certain aspects of the invention, the invention provides an isolated prothymosin alpha polypeptide, wherein the polypeptide comprises an amino acid sequence that is at least about 98% identical to an amino acid sequence of P7-ProTα (SEQ ID NO: 4). In certain aspects of the invention, the invention provides an isolated prothymosin alpha polypeptide, wherein the polypeptide comprises an amino acid sequence that is at least about 95% identical to an amino acid sequence of P7-ProTα (SEQ ID NO: 4). In another aspect, the invention provides an isolated prothymosin alpha polypeptide, wherein the polypeptide comprises an amino acid sequence that is at least about 90% identical to an amino acid sequence of P7-ProTα (SEQ ID NO: 4). In other aspects, the invention provides an isolated prothymosin alpha polypeptide, wherein the polypeptide comprises a functional derivative of P7-ProTα (SEQ ID NO: 4). In yet another aspect, the invention provides an isolated prothymosin alpha polypeptide, wherein the polypeptide comprises a fragment of P7-ProTα (SEQ ID NO: 4).

In certain aspects of the invention, the invention provides an isolated prothymosin alpha polypeptide, wherein the polypeptide comprises an amino acid sequence that is at least about 98% identical to an amino acid sequence of Pseudo4a (SEQ ID NO: 9). In certain aspects of the invention, the invention provides an isolated prothymosin alpha polypeptide, wherein the polypeptide comprises an amino acid sequence that is at least about 95% identical to an amino acid sequence of Pseudo4a (SEQ ID NO: 9). In another aspect, the invention provides an isolated prothymosin alpha polypeptide, wherein the polypeptide comprises an amino acid sequence that is at least about 90% identical to an amino acid sequence of Pseudo4a (SEQ ID NO: 9). In other aspects, the invention provides an isolated prothymosin alpha polypeptide, wherein the polypeptide comprises a functional derivative of Pseudo4a (SEQ ID NO: 9). In yet another aspect, the invention provides an isolated prothymosin alpha polypeptide, wherein the polypeptide comprises a fragment of Pseudo4a (SEQ ID NO: 9).

In certain aspects of the invention, the invention provides an isolated prothymosin alpha polypeptide, wherein the polypeptide comprises an amino acid sequence that is at least about 98% identical to an amino acid sequence of Pseudo4 (SEQ ID NO: 10). In certain aspects of the invention, the invention provides an isolated prothymosin alpha polypeptide, wherein the polypeptide comprises an amino acid sequence that is at least about 95% identical to an amino acid sequence of Pseudo4 (SEQ ID NO: 10). In another aspect, the invention provides an isolated prothymosin alpha polypeptide, wherein the polypeptide comprises an amino acid sequence that is at least about 90% identical to an amino acid sequence of Pseudo4 (SEQ ID NO: 10). In other aspects, the invention provides an isolated prothymosin alpha polypeptide, wherein the polypeptide comprises a functional derivative of Pseudo4 (SEQ ID NO: 10). In yet another aspect, the invention provides an isolated prothymosin alpha polypeptide, wherein the polypeptide comprises a fragment of Pseudo4 (SEQ ID NO: 10).

In certain aspects of the invention, the invention provides an isolated prothymosin alpha polypeptide, wherein the polypeptide comprises an amino acid sequence that is at least about 98% identical to an amino acid sequence of oPTMA (SEQ ID NO: 11). In certain aspects of the invention, the invention provides an isolated prothymosin alpha polypeptide, wherein the polypeptide comprises an amino acid sequence that is at least about 95% identical to an amino acid sequence of oPTMA (SEQ ID NO: 11). In another aspect, the invention provides an isolated prothymosin alpha polypeptide, wherein the polypeptide comprises an amino acid sequence that is at least about 90% identical to an amino acid sequence of oPTMA (SEQ ID NO: 11). In other aspects, the invention provides an isolated prothymosin alpha polypeptide, wherein the polypeptide comprises a functional derivative of oPTMA (SEQ ID NO: 11). In yet another aspect, the invention provides an isolated prothymosin alpha polypeptide, wherein the polypeptide comprises a fragment of oPTMA (SEQ ID NO: 11).

In certain aspects of the invention, the invention provides an isolated prothymosin alpha polypeptide, wherein the polypeptide comprises an amino acid sequence that is at least about 98% identical to an amino acid sequence of ePTMA (SEQ ID NO: 12). In certain aspects of the invention, the invention provides an isolated prothymosin alpha polypeptide, wherein the polypeptide comprises an amino acid sequence that is at least about 95% identical to an amino acid sequence of ePTMA (SEQ ID NO: 12). In another aspect, the invention provides an isolated prothymosin alpha polypeptide, wherein the polypeptide comprises an amino acid sequence that is at least about 90% identical to an amino acid sequence of ePTMA (SEQ ID NO: 12). In other aspects, the invention provides an isolated prothymosin alpha polypeptide, wherein the polypeptide comprises a functional derivative of ePTMA (SEQ ID NO: 12). In yet another aspect, the invention provides an isolated prothymosin alpha polypeptide, wherein the polypeptide comprises a fragment of ePTMA (SEQ ID NO: 12).

In certain aspects of the invention, the invention provides an isolated prothymosin alpha polypeptide, wherein the polypeptide comprises an amino acid sequence that is at least about 98% identical to an amino acid sequence of kPTMA (SEQ ID NO: 13). In certain aspects of the invention, the invention provides an isolated prothymosin alpha polypeptide, wherein the polypeptide comprises an amino acid sequence that is at least about 95% identical to an amino acid sequence of kPTMA (SEQ ID NO: 13). In another aspect, the invention provides an isolated prothymosin alpha polypeptide, wherein the polypeptide comprises an amino acid sequence that is at least about 90% identical to an amino acid sequence of kPTMA (SEQ ID NO: 13). In other aspects, the invention provides an isolated prothymosin alpha polypeptide, wherein the polypeptide comprises a functional derivative of kPTMA (SEQ ID NO: 13). In yet another aspect, the invention provides an isolated prothymosin alpha polypeptide, wherein the polypeptide comprises a fragment of kPTMA (SEQ ID NO: 13).

In one embodiment, the invention provides an isolated prothymosin alpha polypeptide, wherein the polypeptide comprises an amino acid sequence of pProTα (SEQ ID NO: 1). In another embodiment, the invention provides an isolated prothymosin alpha polypeptide, wherein the polypeptide comprises an amino acid sequence of isoA (SEQ ID NO: 2). In other embodiments, the invention provides an isolated prothymosin alpha polypeptide, wherein the polypeptide comprises an amino acid sequence of isoB (SEQ ID NO: 3). In yet another embodiment, the invention provides an isolated prothymosin alpha polypeptide, wherein the polypeptide comprises an amino acid sequence of P7-ProTα (SEQ ID NO: 4). In another embodiment, the invention provides an isolated prothymosin alpha polypeptide, wherein the polypeptide comprises an amino acid sequence of Pseudo4a (SEQ ID NO: 9). In other embodiments, the invention provides an isolated prothymosin alpha polypeptide, wherein the polypeptide comprises an amino acid sequence of Pseudo4 (SEQ ID NO: 10). In another embodiment, the invention provides an isolated prothymosin alpha polypeptide, wherein the polypeptide comprises an amino acid sequence of oPTMA (SEQ ID NO: 11). In other embodiments, the invention provides an isolated prothymosin alpha polypeptide, wherein the polypeptide comprises an amino acid sequence of ePTMA (SEQ ID NO: 12). In yet another embodiment, the invention provides an isolated prothymosin alpha polypeptide, wherein the polypeptide comprises an amino acid sequence of kPTMA (SEQ ID NO: 13).

In other aspects, the invention provides an isolated nucleic acid that encodes a prothymosin alpha polypeptide, wherein the nucleic acid has a nucleotide sequence that is at least about 95% identical to a nucleotide sequence of pProTα (SEQ ID NO: 5). In another aspect, the invention provides an isolated nucleic acid that encodes a prothymosin alpha polypeptide, wherein the nucleic acid has a nucleotide sequence that is at least about 95% identical to a nucleotide sequence of IsoA (SEQ ID NO: 6). In certain aspects, the invention provides an isolated nucleic acid that encodes a prothymosin alpha polypeptide, wherein the nucleic acid has a nucleotide sequence that is at least about 95% identical to a nucleotide sequence of IsoB (SEQ ID NO: 7). In yet another aspect, the invention provides an isolated nucleic acid that encodes a prothymosin alpha polypeptide, wherein the nucleic acid has a nucleotide sequence that is at least about 95% identical to a nucleotide sequence of P7-ProTα (SEQ ID NO: 8). In other aspects, the invention provides an isolated nucleic acid that encodes a prothymosin alpha polypeptide, wherein the nucleic acid has a nucleotide sequence that is at least about 95% identical to a nucleotide sequence of Pseudo4a (SEQ ID NO: 14). In another aspect, the invention provides an isolated nucleic acid that encodes a prothymosin alpha polypeptide, wherein the nucleic acid has a nucleotide sequence that is at least about 95% identical to a nucleotide sequence of Pseudo4 (SEQ ID NO: 15). In certain aspects, the invention provides an isolated nucleic acid that encodes a prothymosin alpha polypeptide, wherein the nucleic acid has a nucleotide sequence that is at least about 95% identical to a nucleotide sequence of oPTMA (SEQ ID NO: 16). In certain aspects, the invention provides an isolated nucleic acid that encodes a prothymosin alpha polypeptide, wherein the nucleic acid has a nucleotide sequence that is at least about 95% identical to a nucleotide sequence of ePTMA (SEQ ID NO: 17). In yet another aspect, the invention provides an isolated nucleic acid that encodes a prothymosin alpha polypeptide, wherein the nucleic acid has a nucleotide sequence that is at least about 95% identical to a nucleotide sequence of kPTMA (SEQ ID NO: 18).

In certain embodiments, the invention provides an isolated nucleic acid that encodes a prothymosin alpha polypeptide, wherein the nucleic acid comprises a nucleotide sequence of pProTα (SEQ ID NO: 5). In another embodiment, the invention provides an isolated nucleic acid that encodes a prothymosin alpha polypeptide, wherein the nucleic acid comprises a nucleotide sequence of IsoA (SEQ ID NO: 6). In certain embodiments, the invention provides an isolated nucleic acid that encodes a prothymosin alpha polypeptide, wherein the nucleic acid comprises a nucleotide sequence of IsoB (SEQ ID NO: 7). In yet another embodiment, the invention provides an isolated nucleic acid that encodes a prothymosin alpha polypeptide, wherein the nucleic acid comprises a nucleotide sequence of P7-ProTα (SEQ ID NO: 8). In certain embodiments, the invention provides an isolated nucleic acid that encodes a prothymosin alpha polypeptide, wherein the nucleic acid comprises a nucleotide sequence of Pseudo4a (SEQ ID NO: 14). In another embodiment, the invention provides an isolated nucleic acid that encodes a prothymosin alpha polypeptide, wherein the nucleic acid comprises a nucleotide sequence of Pseudo4 (SEQ ID NO: 15). In certain embodiments, the invention provides an isolated nucleic acid that encodes a prothymosin alpha polypeptide, wherein the nucleic acid comprises a nucleotide sequence of oPTMA (SEQ ID NO: 16). In another embodiment, the invention provides an isolated nucleic acid that encodes a prothymosin alpha polypeptide, wherein the nucleic acid comprises a nucleotide sequence of ePTMA (SEQ ID NO: 17). In yet another embodiment, the invention provides an isolated nucleic acid that encodes a prothymosin alpha polypeptide, wherein the nucleic acid comprises a nucleotide sequence of kPTMA (SEQ ID NO: 18).

In one embodiment, the invention provides a pharmaceutical composition comprising the polypeptides of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, and a pharmaceutically acceptable carrier. In another embodiment, the invention provides a pharmaceutical composition that comprises a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, and a pharmaceutically acceptable carrier. In another embodiment, the invention provides a pharmaceutical composition comprising a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, and a pharmaceutically acceptable carrier. In yet another embodiment, the invention provides a pharmaceutical composition comprising a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, and a pharmaceutically acceptable carrier.

In certain aspects of the invention, a method for treating or preventing a viral infection is provided which involves administering to a subject in need of such treatment a polypeptide of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, in an amount effective to treat or prevent viral infection. In another aspect of the invention, a method for treating or preventing a viral infection is provided which involves administering to a subject in need of such treatment a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, in an amount effective to treat or prevent viral infection. In others aspects of the invention, a method for treating or preventing a viral infection is provided which involves administering to a subject in need of such treatment a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, in an amount effective to treat or prevent viral infection. In yet another aspect of the invention, a method for treating or preventing a viral infection is provided which involves administering to a subject in need of such treatment a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, in an amount effective to treat or prevent viral infection.

In certain aspects of the invention, the viral infection is caused by Human Immunodeficiency Virus-1 (HIV-1), Human Immunodeficiency Virus-2 (HIV-2), Hepatatis C Virus, Hepatatis B Virus, Hepatatis A Virus, Ebola, Marburg, Dengue viruses, Hemorrhagic Fever Viruses (VHFs), Lassa virus (LASV), Crimean-Congo hemorrhagic fever virus (CCHFV), Rift Valley fever virus (RVFV), and/or yellow fever virus (YFV).

In another aspect of the invention, the viral infection is an HIV-1 and/or HIV-2 infection.

In other embodiments of the invention, a method for potentiating an immune response is provided which involves administering to a subject in need of such treatment a polypeptide of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response is potentiated. In another embodiment of the invention, a method for potentiating an immune response is provided which involves administering to a subject in need of such treatment a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response is potentiated. In other embodiments of the invention, a method for potentiating an immune response is provided which involves administering to a subject in need of such treatment a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response is potentiated. In yet another embodiment of the invention, a method for potentiating an immune response is provided which involves administering to a subject in need of such treatment a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response is potentiated.

In certain embodiments of the invention, the subject has a viral, fungal, bacterial, or parasitic infection, or combinations thereof, in which the present invention potentiates the subject's immune response to the infection(s).

In other embodiments of the invention, a method for potentiating an immune response in a subject undergoing cancer treatment is provided which involves administering to a subject in need of such potentiation a polypeptide of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response to the cancer is potentiated. In another embodiment of the invention, a method for potentiating an immune response in a subject undergoing cancer treatment is provided which involves administering to a subject in need of such potentiation a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response to the cancer is potentiated. In others embodiments of the invention, a method for potentiating an immune response in a subject undergoing cancer treatment is provided which involves administering to a subject in need of such potentiation a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13), or a combination of these peptides, wherein the immune response to the cancer is potentiated. In yet another embodiment of the invention, a method for potentiating an immune response in a subject undergoing cancer treatment is provided which involves administering to a subject in need of such potentiation a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein the immune response to the cancer is potentiated.

In certain embodiments of the invention, the cancer treatment is a chemotherapeutic agent.

In certain aspects of the invention, a method for treating or preventing ischemia is provided which involves administering to a subject in need of such treatment a polypeptide of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, in an amount effective to treat or prevent ischemia. In another aspect of the invention, a method for treating or preventing ischemia is provided which involves administering to a subject in need of such treatment a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, in an amount effective to treat or prevent ischemia. In other aspects of the invention, a method for treating or preventing ischemia is provided which involves administering to a subject in need of such treatment a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, in an amount effective to treat or prevent ischemia. In yet another aspect of the invention, a method for treating or preventing ischemia is provided which involves administering to a subject in need of such treatment a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, in an amount effective to treat or prevent ischemia.

In other embodiments of the invention, a method for inducing interferons is provided which involves administering to a subject in need of interferon induction a polypeptide of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein interferons are induced. In another embodiment of the invention, a method for inducing interferons is provided which involves administering to a subject in need of interferon induction a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein interferons are induced. In other embodiments of the invention, a method for inducing interferons is provided which involves administering to a subject in need of interferon induction a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein interferons are induced. In yet another embodiment of the invention, a method for inducing interferons is provided which involves administering to a subject in need of interferon induction a polypeptide that is a fragment of pProTα (SEQ ID NO: 1), isoA (SEQ ID NO: 2), isoB (SEQ ID NO: 3), and/or P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein interferons are induced.

In certain aspects of the invention, the interferons are type I and/or type III interferons. In other aspects of the invention, the subject in need of type I interferon induction due to a blood disorder, cancer and/or a viral infection. In certain aspects of the invention, the blood disorder is leukemia.

In one embodiment, the invention provides a kit for potentiating an immune response comprising the polypeptides of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and instructions for use. In another embodiment, the invention provides a kit for potentiating an immune response that comprises a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and instructions for use. In another embodiment, the invention provides a kit for potentiating an immune response comprising a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and instructions for use. In yet another embodiment, the invention provides a kit for potentiating an immune response comprising a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and instructions for use.

In one embodiment, the invention provides a pharmaceutical composition comprising the polypeptides of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a chemotherapeutic agent. In another embodiment, the invention provides a pharmaceutical composition that comprises a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a chemotherapeutic agent. In another embodiment, the invention provides a pharmaceutical composition comprising a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a chemotherapeutic agent. In yet another embodiment, the invention provides a pharmaceutical composition comprising a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a chemotherapeutic agent.

In one embodiment, the invention provides a pharmaceutical composition comprising the polypeptides of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, a chemotherapeutic agent, and interferon alpha. In another embodiment, the invention provides a pharmaceutical composition that comprises a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, a chemotherapeutic agent, and interferon alpha. In another embodiment, the invention provides a pharmaceutical composition comprising a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, a chemotherapeutic agent, and interferon alpha. In yet another embodiment, the invention provides a pharmaceutical composition comprising a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, a chemotherapeutic agent, and interferon alpha.

In certain aspects of the invention, the chemotherapeutic agent is dacarbazine, tamoxifen, raloxifene, megestrol, flutamide cyclophosphamide, temozolomide, cisplatin, and/or paclitaxel.

In one embodiment, the invention provides a pharmaceutical composition comprising the polypeptides of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pegylated interferon-alpha2a. In another embodiment, the invention provides a pharmaceutical composition that comprises a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pegylated interferon-alpha2a. In another embodiment, the invention provides a pharmaceutical composition comprising a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pegylated interferon-alpha2a. In yet another embodiment, the invention provides a pharmaceutical composition comprising a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pegylated interferon-alpha2a.

In one embodiment, the invention provides a pharmaceutical composition comprising an antigen, the polypeptides of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pharmaceutically acceptable carrier. In another embodiment, the invention provides a pharmaceutical composition that comprises an antigen, a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pharmaceutically acceptable carrier. In another embodiment, the invention provides a pharmaceutical composition comprising an antigen, a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pharmaceutically acceptable carrier. In yet another embodiment, the invention provides a pharmaceutical composition comprising an antigen, a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, and a pharmaceutically acceptable carrier.

In other embodiments of the invention, a method for inducing RANTES is provided which involves administering to a subject in need of RANTES induction a polypeptide of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein RANTES is induced. In another embodiment of the invention, a method for inducing RANTES is provided which involves administering to a subject in need of RANTES induction a polypeptide that has an amino acid sequence that is at least about 95% identical to an amino acid sequence of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein RANTES is induced. In other embodiments of the invention, a method for inducing RANTES is provided which involves administering to a subject in need of RANTES induction a polypeptide that is a functional derivative of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein RANTES is induced. In yet another embodiment of the invention, a method for inducing RANTES is provided which involves administering to a subject in need of RANTES induction a polypeptide that is a fragment of pProTα (SEQ ID NO: 1), isoA (SEQ ID NO: 2), isoB (SEQ ID NO: 3), P7-ProTα (SEQ ID NO: 4), Pseudo4a (SEQ ID NO: 9), Pseudo4 (SEQ ID NO: 10), oPTMA (SEQ ID NO: 11), ePTMA (SEQ ID NO: 12), kPTMA (SEQ ID NO: 13),or a combination of these peptides, wherein RANTES is induced.

### BRIEF DESCRIPTION OF THE DRAWINGS

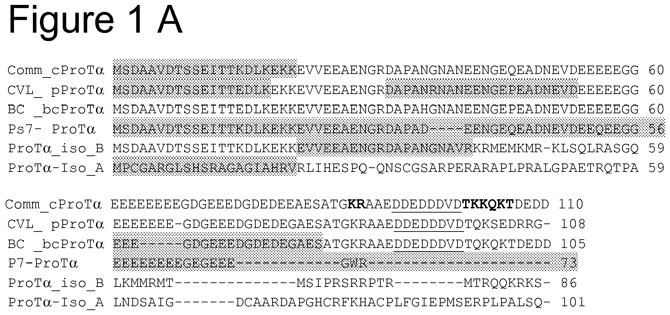

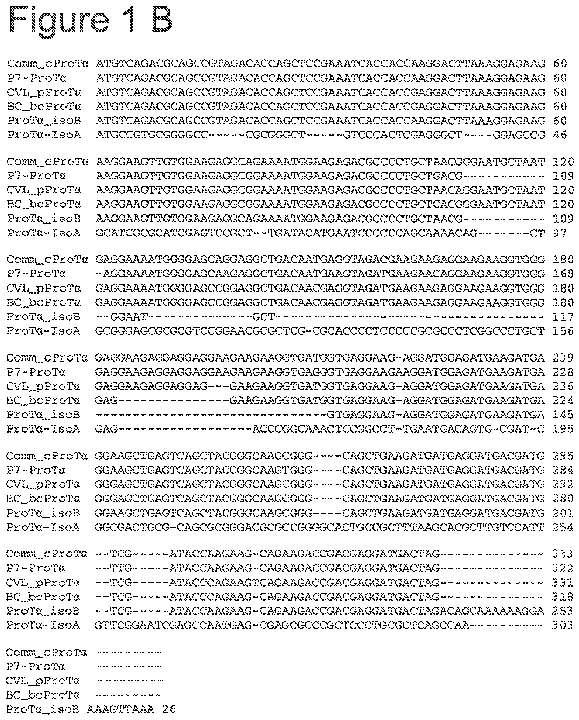

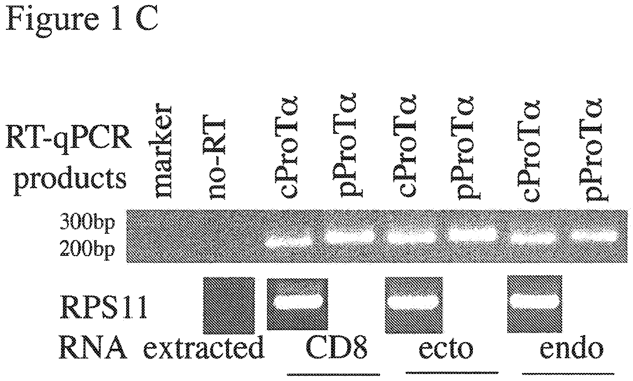

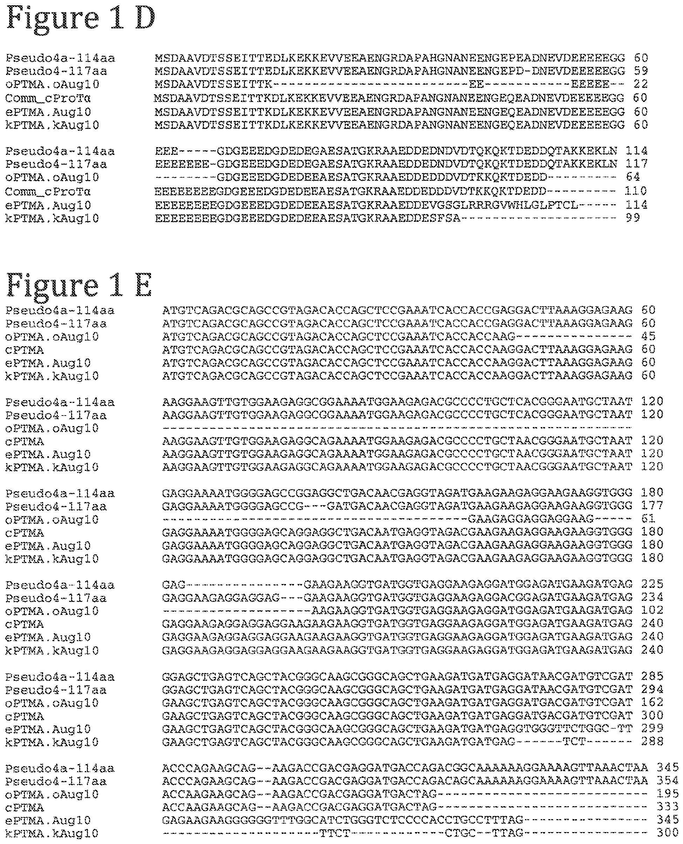

Figure 1 shows exemplary protein and nucleotide sequences of nine novel ProTα variants aligned with common ProTα (cProTα) and/or breast cancer variant (bcProTα) and demonstrates expression of novel pseudogene ProTα (pProTα) variant.
(A) Amino acid sequence alignment of cProTα, bcProTα, and four novel ProTα variants (pProTα, isoA, isoB, and P7-ProTα). Peptides identified by mass spectrometry are highlighted.
(B) Nucleotide sequence alignment of cProTα, bcProTα, and four novel ProTα variants (pProTα, isoA, isoB, and P7-ProTα). RT-PCR of pseudogene-(pProTα) and common- (cProTα) ProTα genes was performed using the same forward primer, but gene-specific reverse primers.
(C) Expression of pProTα mRNA is detected. Agarose gel electrophoresis of RT-PCR products revealed amplicon sizes and expression levels of pProTα and cProTα. Lane 1 contains a faintly visible molecular weight marker; lane 2, labeled no-RT, contains extracted RNA that did not undergo amplification; lanes 3 and 4 contain RNA extracted from CD8⁺ cells with qPCR products from (3) cProTα and (4) cProTα; lanes 5 and 6 contain extracted RNA from ectocervical cells with qPCR products from (5) cProTα and (6) cProTα; lanes 7 and 8 contain extracted RNA from endocervical cells with qPCR products from (7) cProTα and (8) cProTα. Ribosomal protein S11 (RPS11) served as a housekeeping reference gene in all three cell lines.
(D) Amino acid sequence alignment of cProTα and five novel ProTα variants (Pseudo4a, Pseudo4, oPTMA, ePTMA, and kPTMA). These five variants are variants of prothymosin alpha protein. Pseudo4a and Pseudo 4 are derived from genes considered pseudogenes. oPTMA, ePTMA, and kPTMA are splice variants of single known pothymosin alpha protein.
(E) Nucleotide sequence alignment of cProTα, and five novel ProTα variants (Pseudo4a, Pseudo4, oPTMA, ePTMA, and kPTMA).

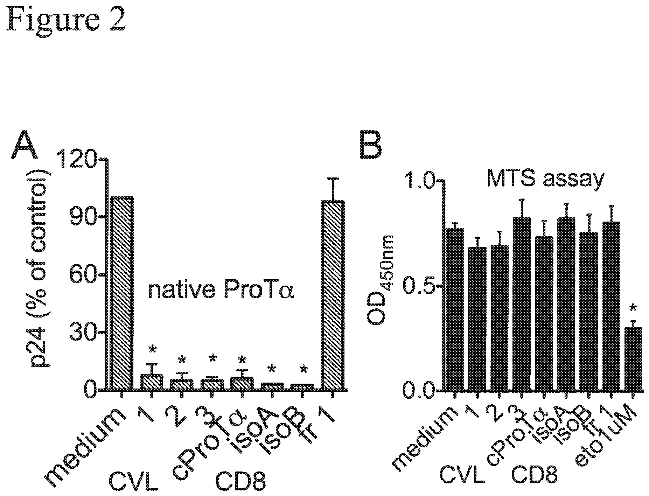

Figure 2 shows that native ProTα variants, pProTα, isoA-ProTα and isoB-ProTα, have potent anti-HIV activity with no detectable cytotoxic effect. All experiments were performed at least three times. Bars marked with an asterisk were considered statistically significant (p < 0.05).
(A) ELISA results show that infected human macrophages treated with ProTα variants (3ng/ml) and cProTα (200 ng/ml) have suppressed HIV-1 replication, as measured by levels of HIV p24 antigen, compared to macrophages treated with fraction 1 or medium alone. Values are shown as a percent of p24 antigen measured in control samples (untreated human macrophages infected with HIV_{BaL}).
(B) The MTS assay demonstrates that none of the treatments in (A) induced cellular toxicity (a DNA-damaging agent, etoposide, was used as a positive control).

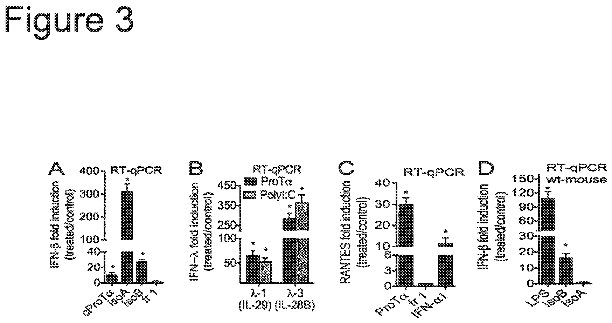

Figure 3 shows that native ProTα variants isoA and isoB induce mRNA expression of innate inhibitors of HIV-1 replication: type I IFN (IFN-β), type III IFNs (λ1 and λ3) and RANTES in primary human macrophages. All mRNA transcripts were measured by RT-qPCR where fold change in mRNA expression of treated cells was calculated as compared with control medium after normalization to the human ribosomal protein (RPS11) control gene in (A), (B), (C), and D. Each experiment was done at least three times. Bars marked with an asterisk were considered statistically significant (p< 0.05).
(A) Type I IFN-β mRNA expression was significantly enhanced by treatment of primary human macrophages with native cProTα, isoA-ProTα or isoB-ProTα for 2 hours.
(B) Type III IFN (λ1 and λ3) mRNA expression was significantly enhanced by treatment of primary human macrophages with recombinant human ProTα or poly I:C (a chemical analog of double stranded RNA) used as a positive control for 4 hours.
(C) RANTES mRNA expression was significantly increased at 4h post treatment of primary human macrophages with human ProTα (10 ng/ml-NO 200ng/ml) or human IFN-α1 (10 ng/ml) (positive control).
(D) Type I IFN-β mRNA expression in wild type mouse macrophages did not conserve the Type I IFN-β mRNA expression pattern detected in human macrophages in (A). In mouse macrophages, levels of mRNA were mildly enhanced by isoB-ProTα and not significantly changed by isoA-ProTα treatment for 2 hours.

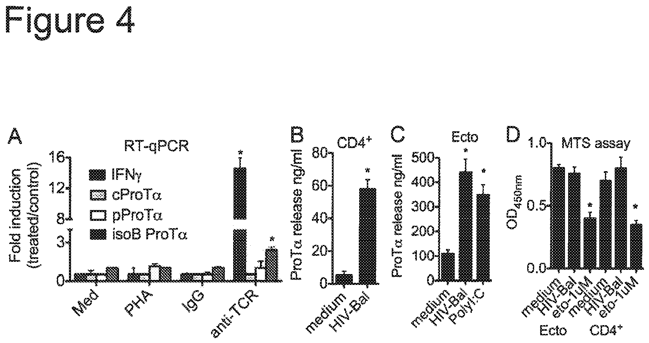

Figure 4 shows induction of mRNA of ProTα variants by TCR stimulation of CD8⁺ T-cells and release of ProTα from HIV_{BAL} infected CD4⁺ T cells or ectocervical cells stimulated with poly I:C. Each experiment was done at least three times. Bars marked with an asterisk were considered statistically significant (p< 0.05).
(A) The mRNA expression of ProTα variants was triggered by T-cell receptor (TCR) stimulation of CD8⁺ T-cells. Primary human CD8⁺ T cells were stimulated with PHA or with beads conjugated with anti-CD3 antibody for 24 hours. As controls, samples were incubated with media containing unconjugated IgG or media alone. Fold change in mRNA expression of treated cells was calculated as compared with control medium after normalization to the human ribosomal protein (RPS11) control gene. Expression of human IFNγ was monitored as a positive control for TCR induction.
(B) Release of ProTα protein was enhanced from primary CD4⁺ T cells infected with HIV_{BaL} at a MOI of 0.1. Supernatant was collected and used in a ProTα ELISA assay.
(C) Release of ProTα protein was enhanced from ectocervical cells treated with poly I:C (100ug/ml) or infected with HIV_{BaL} at MOI 0.1 for 24 hours. Supernatant was collected and used for ProTα ELISA assay.
(D) MTS assay demonstrates that neither infection with HIV_{BaL} nor mock infection for 24 hours induces cellular toxicity in CD4⁺ cells or ectocervical cells (a DNA-damaging agent, etoposide, was used as a positive control).

Figure 5 shows the mass spectrometry of the unique peptide sequences of ProTα variants isoB and P7 from CD8 sups and CVL-derived active fractions.

Figure 6 shows the native genetic variants of ProTα IsoB and p7 suppress HIV replication in primary human macrophages.
(A) The anti-HIV activity of chromatographic peaks containing native genetic variants of ProTα (95% pure for the indicated proteins). Primary human macrophages were infected with HIV_{BaL} at MOI 0.1 for 2 h followed by washes with PBS and treated with volumes equivalent to 3 ng/ml protein of the indicated ProTα variant-containing peaks. ELISA for the presence of HIV p24 antigen was done at day 7 post infection.
(B) MTS cytotoxicity assay was performed on primary human macrophages treated with native genetic variants of ProTα v (3 ng/ml) or as a positive control 2 µM etoposide (DNA-damaging agent) for 7 days. MTS assay was done according to manufacturer's instruction.

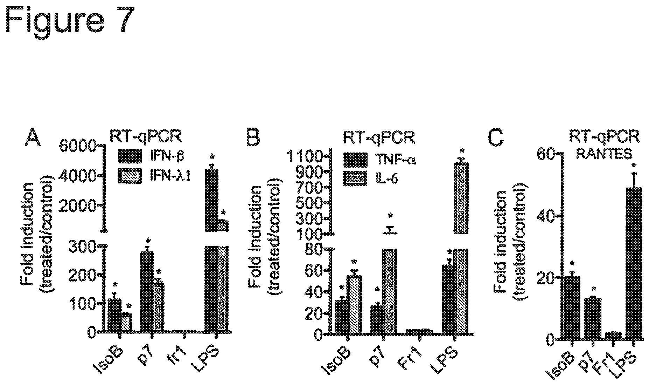

Figure 7 shows that the native genetic variants of ProTα IsoB and p7 induce mRNA expression of type I, type III IFNs and RANTES but not the fraction derived from the same purification (Fr 1) containing the equal amount of the protein. RT-qPCR was performed on RNA extracted from treated and control cells with gene specific primers. All mRNA transcripts were measured by RT-qPCR where fold change in mRNA expression of treated cells was calculated as compared with control medium after normalization to the ribosomal protein (RPS11) control gene. Each experiment was done at least three times. Data represent mean ± SD. Student's *t* test was used to compare means of treated versus control samples. Bars marked with an asterisk were considered statistically significant (p< 0.05).
(A) IFN-β and type III IFN mRNA induction.
(B) TNF-α and IL-6 mRNA induction.
(C) RANTES mRNA induction at 2 h post treatment of primary human macrophages with 3 ng/ml of different ProTα variants.

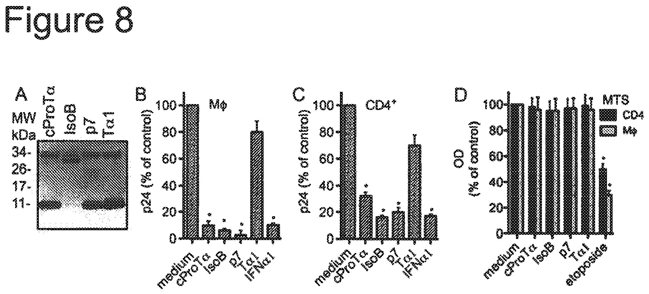

Figure 8 shows that recombinant genetic variants of ProTα: cProTα, IsoB and p7 suppress HIV replication. Primary human macrophages or CD4+ T cells were infected with HIV_{BaL} at MOI 0.1 for 2 h followed by washes with PBS and treated with 200 ng/ml of the indicated ProTα variants. ELISA for the presence of HIV p24 antigen was done at day 7 post infection. Data represent mean ± SD. Student's *t* test was used to compare means of treated versus control samples. Bars marked with an asterisk were considered statistically significant (p< 0.05).
(A) Western blotting analyses of recombinant genetic variants of ProTα; cProTα, p7-, IsoB and N-terminal peptide (Tα1).
(B) Anti-HIV activity of recombinant genetic variants of ProTα in macrophages.
(C) Anti-HIV activity of recombinant genetic variants of ProTα in primary human CD4+ T cells.
(D) MTS cytotoxicity assay was performed on primary human macrophages treated with ProTα variants (200 ng/ml) or as a positive control 2 µM

etoposide (DNA-damaging agent) for 7 days. MTS assay was done according to manufacturers' instruction.

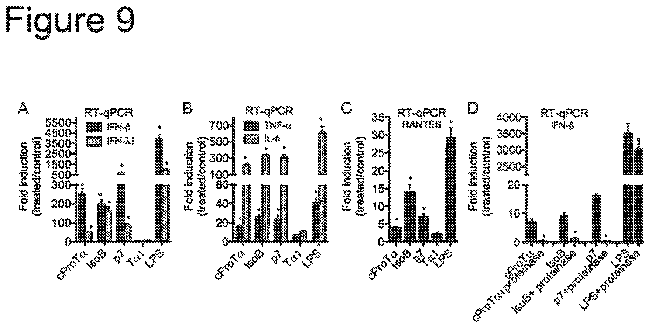

Figure 9 shows that recombinant genetic variants of ProTα: cProTα, IsoB and p7 induce mRNA expression of type I, type III IFNs and RANTES and that this activity is independent of LPS. Two h post treatment of primary human macrophages with 200 ng/ml of different ProTα variants. RT-qPCR was performed as described in Fig. 2. Each experiment was done at least three times. Data represent mean ± SD. Student's *t* test was used to compare means of treated versus control samples. Bars marked with an asterisk were considered statistically significant (p< 0.05).
(A) IFN-β and type III IFN mRNA induction.
(B) TNF-α and IL-6 mRNA induction.
(C) RANTES mRNA induction.
(D) IFN-β mRNA by different genetic variants of ProTα (20 ng/ml) with and without proteinase V8 digestion.

### DETAILED DESCRIPTION

This invention generally relates to novel human ProTα polypeptide variants that have cell mediated immunity activity, but lack the pro-proliferative activity typically associated with ProTα. Cell-mediated immune responses, a fundamental component of adaptive immunity, are critical for mediating an organism's defense against disease. Upregulation of cellular immune responses generally results in a more rapid or thorough clearance of a virus, bacterium, fungus, parasite, cancer, ischemia, myeloproliferative blood disorders or some combination thereof.

The invention also relates to the use of these novel polypeptides for the treatment, prevention, or inhibition of viral infections, such as HIV-1, bacterial infections, fungal infections, cancer, ischemia, and blood disorders. The invention is further directed to compositions comprising ProTα variants and to methods for the use of such compositions in the prevention and/or treatment of viral infections, bacterial infection, fungal infection, cancer, ischemia, and blood disorders.

### Overview

Human ProTα protein is a small 12.5-kDa, strongly acidic polypeptide that is localized intracellularly and extracellularly. Across species, it has a highly conserved primary structure, but lacks a uniform secondary structure. ProTα is abundantly expressed in a wide array of cell types, in a manner similar to other ubiquitous proteins such as ribosomal proteins, which suggests an essential function for this protein. The intracellular function of ProTα is linked to cell proliferation, cell cycle regulation and transcription, as it is overexpressed in various types of cancer (Tripathi, SC et al, PLoS One 6(5): e19213, 2011). As an extracellular protein, ProTα exhibits cytokine-like properties and enhances cell-mediated immunity.

The present invention is based on the discovery of the *in vivo* expression of novel human ProTα variants. The identification of the novel variants is surprising because although ten ProTα gene family member were known to exist, only one form of the protein and corresponding mRNA transcript had been detected (referred to as common-ProTα, or cProTα). One new variant, isolated from cervicolavage (CVL) fluid, was long considered to be an unexpressed pseudogene of ProTα (referred to as pseudogene-ProTα, pProTα). Two other novel variants, isolated from CD8⁺ cell conditioned media, are splice variants of cProTα (referred to as isoform A and isoform B or isoA and isoB), which have also never been previously detected *in vivo.* Further characterization of these three novel ProTα variants revealed that they lack a functional nuclear localization signal (NLS) and consequently, they are localized extracellularly, in contrast to an intracellular cProTα.

### Nuclear Localization Signals (NLSs)

A "nuclear localization signal" or "NLS" as used herein, generally refers to a short, positively charged (basic) amino acid sequence. The NLS marks a protein for import into the nucleus by way of nuclear transport. An exemplary NLS in human ProTα occurs in the C-terminal domain of the protein. While studies have demonstrated that cProTα's NLS is located at amino acid residues 82-109, it has been shown that cProTα actually possesses a bipartite nuclear localization signal (KR amino acid residues at 87 and KKQK at 101) because mutation in both domains impairs nuclear localization *(See* Mosoian, A. 2011 Future Med Chem 3(9):1199-1208).

### Isolated Prothymosin Alpha Variants

### Peptides

As used herein, the singular forms "a," "an" and "the" include plural references unless the content clearly dictates otherwise.

The term "about", as used here, refers to +/- 10% of a value.

As used herein, the term "peptide" refers to an oligomer of at least two contiguous amino acids, linked together by a peptide bond, and not greater than fifty amino acids. As used herein, the term "polypeptide" refers to an oligomer of at least fifty amino acids.

According to the present invention, the terms "modification" and "mutation" can be used interchangeably, particularly with regard to the modifications/mutations to the primary amino acid sequences of a protein or peptide (or nucleic acid sequences) described herein. The term "modification" can also be used to describe post-translational modifications to a protein or peptide including, but not limited to, methylation, farnesylation, carboxymethylation, geranyl geranylation, glycosylation, phosphorylation, acetylation, myristoylation, prenylation, palmitation, and/or amidation. Modification can also include the cleavage of a signal peptide, or methionine, or other portions of the peptide that require cleavage to generate the mature peptide. Modifications can also include, for example, complexing a protein or peptide with another compound. Such modifications can be considered to be mutations, for example, if the modification is different than the post-translational modification that occurs in the natural, wild-type protein or peptide.

As used herein, the terms "homologue" or "variants" are used to refer to a protein or peptide which differs from a naturally occurring protein or peptide (i.e., the "prototype" or "wild-type" protein) by minor modifications to the naturally occurring protein or peptide, but which maintains the basic protein and side chain structure of the naturally occurring form. Such changes include, but are not limited to: changes in one or a few amino acid side chains; changes one or a few amino acids, including deletions (e.g., a truncated version of the protein or peptide), insertions and/or substitutions; changes in stereochemistry of one or a few atoms; and/or minor derivatizations, including but not limited to: methylation, glycosylation, phosphorylation, acetylation, myristoylation, prenylation, palmitation, amidation and/or addition of glycosylphosphatidyl inositol. A homologue or variant can have either enhanced, decreased, or substantially similar properties as compared to the naturally occurring protein or peptide. A homologue or variant can include an agonist of a protein or an antagonist of a protein.

In certain embodiments, a ProTα variant of the invention comprises a polypeptide having an amino acid sequence corresponding to one of the following sequences that is at least about 100% identical, at least about 99% identical, at least about 98% identical, at least about 97% identical, at least about 96% identical, at least about 95% identical, at least about 90%, at least about 85% identical, at least about 80% identical, or at least about 75% identical to the following sequences:
**pProTα (SEQ ID NO. 1):** 
MSDAAVDTSSEITTEDLKEKKEVVEEAENGRDAPANRNANEENGEPEADNEVDEEE
EEGGEEEEEEEEGDGEEEDGDEDEGAESATGKRAAEDDEDDDVDTQKSEDRRG
**IsoA (SEQ ID NO. 2):** 
MPCGARGLSHSRAGAGIAHRVRLIHESPQQNSCGSARPERARAPLPRALGPAETRQT
PALNDSAIGDCAARDAPGHCRFKHACPLFGIEPMSERPLPALSQ
**IsoB (SEQ ID NO. 3):** 
MSDAAVDTSSEITTKDLKEKKEVVEEAENGRDAPANGNAVRKRMEMKMRKLSQLR
ASGQLKMMRMTMSIPRSRRPTRMTRQQKRKS
**P7-ProTα (SEQ ID NO. 4):**
**Pseudo4a (SEQ ID NO. 9):**
**Pseudo4 (SEQ ID NO. 10):** 
MSDAAVDTSSEITTEDLKEKKEVVEEAENGRDAPAHGNANEENGEPDDNEVDEEEE
EGGEEEEEEEEGDGEEEDGDEDEGAESATGKRAAEDDEDNDVDTQKQKTDEDDQTA
KKEKLN
**oPTMA (SEQ ID NO. 11):** 
MSDAAVDTSSEITTKEEEEEEEGDGEEEDGDEDEEAESATGKRAAEDDEDDDVDTK
KQKTDEDD
**ePTMA (SEQ ID NO. 12):** 
MSDAAVDTSSEITTKDLKEKKEVVEEAENGRDAPANGNANEENGEQEADNEVDEEE
EEGGEEEEEEEEGDGEEEDGDEDEEAESATGKRAAEDDEVGSGLRRRGVWHLGLPT
CL
**kPTMA (SEQ ID NO. 13):**

MSDAAVDTSSEITTKDLKEKKEVVEEAENGRDAPANGNANEENGEQEADNEVDEEE
EEGGEEEEEEEEEGDGEEEDGDEDEEAESATGKRAAEDDESFSA

The polypeptide of the invention can be synthesized according to Merrifield solid-phase synthesis techniques (Kotler et al., Proc. Natl. Acad. Sci. 85:4185-4189, 1985; Barany et al., in Gross et al., eds., The Peptides, Vol. 2, Academic Press, 1980) or other techniques of peptide synthesis known to those skilled in the art. After cleavage and deprotection, synthetic peptides or polypeptides can be purified by, for example, gel filtration, chromatography, and any reverse-phase column/HPLC system known to those skilled in the art. Polypeptides derived from the invention may also be produced by chemical or enzymatic digestion of the full-length protein using techniques that are known to those skilled in the art.

A polypeptide may be recovered by purification from a cell line expressing such a polypeptide, using standard techniques for protein purification which are known to those skilled in the art, including, but not limited to, size fractionation, ion-exchange chromatography, and reverse-phase chromatography. The amino acid sequences of the polypeptides can be confirmed and identified by amino acid composition analysis as well as manual and automated Edman degradation and determination of each amino acid, HPLC analysis, or mass spectrometry

As used herein, reference to an "isolated protein" or "isolated polypeptide" in the present invention, includes full-length proteins and their glycosylated or otherwise modified forms, fusion proteins, or any fragment or homologue or variant of such a protein. More specifically, an isolated protein according to the present invention, is a protein (including a polypeptide or peptide) that has been removed from its natural milieu (*i.e.,* that has been subject to human manipulation) and can include purified proteins, partially purified proteins, recombinantly produced proteins, synthetically produced proteins, proteins complexed with lipids, soluble proteins, and isolated proteins associated with other proteins, for example. As such, "isolated" does not reflect the extent to which the protein has been purified.

### Nucleic acid

As used herein, the term "nucleic acid" or "polynucleotide" refers to an oligomer of at least two contiguous nucleotides, linked together by a phosphodiester bond. A nucleic acid can also be referred to as DNA, RNA or derivatives of either DNA or RNA, including cDNA.

In accordance with the present invention, an "isolated nucleic acid" molecule is a nucleic acid molecule (polynucleotide) that has been removed from its natural milieu (*i. e.,* that has been subject to human manipulation) and can include DNA, RNA, or derivatives of either DNA or RNA, including cDNA. As such, "isolated" does not reflect the extent to which the nucleic acid molecule has been purified. The phrase "nucleic acid sequence" primarily refers to the sequence of nucleotides on the nucleic acid molecule that is capable of encoding a protein. An isolated nucleic acid molecule of the present invention can be isolated from its natural source or produced using recombinant DNA technology (e.g., polymerase chain reaction (PCR) amplification, cloning) or chemical synthesis. Isolated nucleic acid molecules can include, for example, genes, natural allelic variants of genes, coding regions or portions thereof, and coding and/or regulatory regions modified by nucleotide insertions, deletions, substitutions, and/or inversions in a manner such that the modifications do not substantially interfere with the nucleic acid molecule's ability to encode a protein of the present invention or to form stable hybrids under stringent conditions with natural gene isolates.

In certain embodiments, a ProTα variant of the invention comprises a nucleic acid sequence corresponding to one of the following sequences that is at least about 100% identical, at least about 99% identical, at least about 98% identical, at least about 97% identical, at least about 96% identical, at least about 95% identical, at least about 90%, at least about 85% identical, at least about 80% identical, or at least about 75% identical to the following sequences:
**pProTα (SEQ ID NO. 5):** 
atgtcagacgcagccgtagacaccagctccgaaatcaccaccgaggacttaaaggagaagaaggaagttgtggaagaggcggaaa
atggaagagacgcccctgctaacaggaatgctaatgaggaaaatggggagccggaggctgacaacgaggtagatgaagaagagg
aagaaggtggggaggaagaggaggaggaagaaggtgatggtgaggaagaggatggagatgaagatgagggagctgagtcagct
acgggcaagcgggcagctgaagatgatgaggatgacgatgtcgatacccagaagtcagaagaccgacgaggatgactag
**IsoA (SEQ ID NO. 6):** 
atgccgtgcggggcccgcgggctgtcccactcgagggctggagccggcatcgcgcatcgagtccgcttgatacatgaatcccccca
gcaaaacagctgcgggagcgcgcgtccggaacgcgctcgcgcacccctcccccgcgccctcggccctgctgagacccggcaaac
tccggccttgaatgacagtgcgatcggcgactgcgcagcgcgggacgcgccggggcactgccgctttaagcacgcttgtccattgtt
cggaatcgagccaatgagcgagcgcccgctccctgcgctcagccaa
**IsoB (SEQ ID NO. 7):** 
atgtcagacgcagccgtagacaccagctccgaaatcaccaccaaggacttaaaggagaagaaggaagttgtggaagaggcagaaa
atggaagagacgcccctgctaacgggaatgctgtgaggaagaggatggagatgaagatgaggaagctgagtcagctacgggcaag
cgggcagctgaagatgatgaggatgacgatgtcgataccaagaagcagaagaccgacgaggatgactagacagcaaaaaaggaa
aagttaaa
**P7-ProTα (SEQ ID NO. 8):** 
atgtcagacgcagccgtagacaccagctccgaaatcaccaccaaggacttaaaggagaagaaggaagttgtggaagaggcggaaa
atggaagagacgcccctgctgacgaggaaaatggggagcaagaggctgacaatgaagtagatgaagaacaggaagaaggtggg
gaggaagaggaggaggaagaagaaggtgagggtgaggaagaaggatggagatgaagatgaggaagctgagtcagctaccggc
aagtgggcagctgaagatgatgaggatgacgatgttgataccaagaagcagaagaccgacgaggatgactag
**Pseudo4a (SEQ ID NO. 14):** 
atgtcagacgcagccgtagacaccagctccgaaatcaccaccgaggacttaaaggagaagaaggaagttgtggaagaggcggaaa
atggaagagacgcccctgctcacgggaatgctaatgaggaaaatggggagccggaggctgacaacgaggtagatgaagaagagg
aagaaggtggggaggaagaaggtgatggtgaggaagaggatggagatgaagatgagggagctgagtcagctacgggcaagcgg
gcagctgaagatgatgaggataacgatgtcgatacccagaagcagaagaccgacgaggatgaccagacggcaaaaaaggaaaag
ttaaactaa
**Pseudo4 (SEQ ID NO. 15):** 
atgtcagacgcagccgtagacaccagctccgaaatcaccaccgaggacttaaaggagaagaaggaagttgtggaagaggcggaaa
atggaagagacgcccctgctcacgggaatgctaatgaggaaaatggggagccggatgacaacgaggtagatgaagaagaggaag
aaggtggggaggaagaggaggaggaagaaggtgatggtgaggaagaggacggagatgaagatgagggagctgagtcagctac
gggcaagcgggcagctgaagatgatgaggataacgatgtcgatacccagaagcagaagaccgacgaggatgaccagacagcaaa
aaaggaaaagttaaactaa
**oPTMA (SEQ ID NO. 16):** 
atgtcagacgcagccgtagacaccagctccgaaatcaccaccaaggaagaggaggaggaagaagaaggtgatggtgaggaagag
gatggagatgaagatgaggaagctgagtcagctacgggcaagcgggcagctgaagatgatgaggatgacgatgtcgataccaaga
agcagaagaccgacgaggatgactag
**ePTMA (SEQ ID NO. 17):** 
atgtcagacgcagccgtagacaccagctccgaaatcaccaccaaggacttaaaggagaagaaggaagttgtggaagaggcagaaa
atggaagagacgcccctgctaacgggaatgctaatgaggaaaatggggagcaggaggctgacaatgaggtagacgaagaagagg
aagaaggtggggaggaagaggaggaggaagaagaaggtgatggtgaggaagaggatggagatgaagatgaggaagctgagtca
gctacgggcaagcgggcagctgaagatgatgaggtgggttctggcttgagaagaagggggggtttggcatctgggtctccccacctgc
ctttag
**kPTMA (SEQ ID NO. 18):**

atgtcagacgcagccgtagacaccagctccgaaatcaccaccaaggacttaaaggagaagaaggaagttgtggaagaggcagaaa
atggaagagacgcccctgctaacgggaatgctaatgaggaaaatggggagcaggaggctgacaatgaggtagacgaagaagagg
aagaaggtggggaggaagaggaggaggaagaagaaggtgatggtgaggaagaggatggagatgaagatgaggaagctgagtca
gctacgggcaagcgggcagctgaagatgatgagtctttctctgcttag

### Preparation and Purification of ProTα Variants

ProTα variant proteins may be prepared in a variety of ways, according to known methods. Polynucleotides encoding ProTα variants may be produced by chemical synthesis, e.g., by the phosphoramidite method described by Beaucage and Caruthers (1981) Tetra. Letts., 22:1859-1862 or the triester method according to Matteuci et al. (1981) J. Am. Chem. Soc., 103: 3185, and may be performed on commercial automated oligonucleotide synthesizers. A double-stranded fragment may be obtained from the single stranded product of chemical synthesis either by synthesizing the complementary strand and annealing the strands together under appropriate conditions or by adding the complementary strand using DNA polymerase with an appropriate primer sequence.

Naturally occurring ProTα variant proteins may be purified from appropriate sources (e.g., CD8⁺ cells, CD4 cells, and cells isolated from the body (e.g., cervicovaginal lavage fluids which can potentially contain epithelial, dendritic, macrophage, B-cells, and additional cells found in the female reproductive track). ProTα variant proteins may also be generated using *in vitro* transcription and translation systems. For example, a cDNA or gene may be cloned into an appropriate *in vitro* transcription vector, such as pSP64 or pSP65 for *in vitro* transcription, followed by cell-free translation in a suitable cell-free translation system, such as wheat germ or rabbit reticulocyte lysates. *In vitro* transcription and translation systems are commercially available, e.g., from Promega Biotech, Madison, Wis. or BRL, Rockville, Md.

In another embodiment, larger quantities of ProTα variants may be produced by expression in a suitable prokaryotic or eukaryotic system. For example, part or all of a DNA molecule, such as a cDNA of the variants, may be inserted into a plasmid vector adapted for expression in a bacterial cell, such as *E. coli.* Such vectors comprise regulatory elements necessary for expression of the DNA in a host cell (e.g. *E. coli*) positioned in such a manner as to permit expression of the DNA in the host cell. Such regulatory elements required for expression include promoter sequences, transcription initiation sequences and, optionally, enhancer sequences which are operably linked.

As used herein, the term "operably linked" refers to a regulatory sequence capable of mediating the expression of a coding sequence and which are placed in a DNA molecule (e.g., an expression vector) in an appropriate position relative to the coding sequence so as to effect expression of the coding sequence. This same definition is sometimes applied to the arrangement of coding sequences and transcription control elements (e.g. promoters, enhancers, and termination elements) in an expression vector. This definition is also sometimes applied to the arrangement of nucleic acid sequences of a first and a second nucleic acid molecule wherein a hybrid nucleic acid molecule is generated.

ProTα variant polypeptides or nucleic acids produced by gene expression in a recombinant prokaryotic or eukaryotic system may be purified according to methods known in the art. In one embodiment, a commercially available expression/secretion system can be used, whereby the recombinant protein is expressed and purified. In one embodiment the protein is secreted from the host cell, to be easily purified from the surrounding medium. If expression/secretion vectors are not used, an alternative approach involves purifying the protein by affinity separation, such as by immunological interaction with antibodies that bind specifically to the recombinant protein or nickel columns for isolation of recombinant proteins tagged with 6-8 histidine residues at their N-terminus or C-terminus. Alternative tags may comprise the FLAG epitope or the hemagglutinin epitope. Such methods are commonly used by skilled practitioners.

The term "tag", "tag sequence" or "protein tag" refers to a chemical moiety, either a nucleotide, oligonucleotide, polynucleotide or an amino acid, peptide or protein or other chemical, that when added to another sequence, provides additional utility or confers useful properties to the sequence, particularly with regard to methods relating to the detection or isolation of the sequence. Thus, for example, a homopolymer nucleic acid sequence or a nucleic acid sequence complementary to a capture oligonucleotide may be added to a primer or probe sequence to facilitate the subsequent isolation of an extension product or hybridized product. In the case of protein tags, histidine residues (e.g., 4 to 8 consecutive histidine residues) may be added to either the amino- or carboxy-terminus of a protein to facilitate protein isolation by chelating metal chromatography. Alternatively, amino acid sequences, peptides, proteins or fusion partners representing epitopes or binding determinants reactive with specific antibody molecules or other molecules (e.g., flag epitope, c-myc epitope, transmembrane epitope of the influenza A virus hemaglutinin protein, protein A, cellulose binding domain, calmodulin binding protein, maltose binding protein, chitin binding domain, glutathione S-transferase, and the like) may be added to proteins to facilitate protein isolation by procedures such as affinity or immunoaffinity chromatography. Chemical tag moieties include such molecules as biotin, which may be added to either nucleic acids or proteins and facilitates isolation or detection by interaction with avidin reagents, and the like. Numerous other tag moieties are known to, and can be envisioned by, the trained artisan, and are contemplated to be within the scope of this definition.

### Functional Characterization of ProTα Variants

ProTα variants of the invention are characterized by an ability to decrease or prevent viral, bacterial, or fungal replication in active infections *in vivo* or in a cellular model system, including primary or immortalized cells. In other embodiments, ProTα variants of the invention are characterized by an ability to decrease or prevent cancer, ischemia or blood disorders *in vivo* or in a cellular model system, including primary or immortalized cells. Exemplary cell lines which can be used for ProTα studies on infected cells are preferably those that are susceptible to such infections or diseases. Such cells include, for example, peripheral blood lymphocytes (PBL), CD4+ cells, macrophages, dendritic cells, and epithelial cells, such as epithelial cells for influenza virus. ProTα variants may also be characterized by their effects in altering, reducing or eliminating disease or the replication or infectivity of a virus, bacteria or fungus. In other embodiments, ProTα variants may also be characterized by their effects in altering, reducing or preventing (for example, it could be use by women as a cream before sex to protect them from HIV infected partner) HIV-1 infection. Where ProTα variants are incubated with infected or diseased cells, the production of infectious progeny or the increase in diseased cells is determined relative to control experiments without the variant.

ProTα variants of the invention can be characterized in tissue culture models of viral infection using cells having any lentiviral or retroviral infection, including, but not limited to, those resulting from HIV-1, HIV-2, simian immunodeficiency virus (SIV), feline immunodeficiency virus (FIV), bovine immunodeficiency virus (BIV), and visna virus infections, and all strains and isolates thereof. Specific HIV strains that have tropism for certain cell types can be used, including the macrophage-tropic HIV Ba-1, and the T-tropic HIV IIIB and MN. In general, isolates can include lymphotropic and macrophage-tropic strains, primary strains derived from blood cells or tissues, and North American, European, African and Asian isolates.

Primary cells or cell lines which can be used for inhibitor studies of virus-infected cells are preferably those that are susceptible to such viral infection. Such cells include, for example, peripheral blood lymphocytes (PBL), especially CD4+ cells, and macrophages, dendritic, and epithelial cells, such as epithelial cells for influenza virus.

Assay systems which employ a vector-delivered full or partial viral, bacterial or fungal genome into a eukaryotic cell can be used to simulate virion, bacteria or fungal production, respectively, and such cells can be used in the characterization of ProTα variants. Further assay systems which employ a vector encoding full or partial cancer, ischemic or myeloproliferative signaling pathway, which is then delivered into a eukaryotic cell, can be used to simulate cancer, ischemia or blood disorders, respectively, and such cells can be used in the characterization of ProTα variants.

### Markers to assess effects of ProTα variants

To assess whether ProTα variants reduce or eliminate the generation of diseased cell proliferation or infectious progeny, the quantity and type of diseased cells or infectious progeny are assayed at suitable times post-infection. Evidence of microscopically observed spread of infection or disease and cytopathic effect can provide an assessment as to whether infectious progeny are being generated or diseased cells are proliferating. For example, increased amounts of the p24 capsid protein can provide an assessment as to whether infectious HIV progeny are being generated.

The assessment of progeny infectivity or diseased cell proliferation may be determined further by recovery of infected or diseased cells and co-cultivation with suitable cells (e.g., PBL, macrophages, or dendritic cells) or by the recovery of supernatant from the infected or diseased cells and cell-free infection of suitable cells. For example, the rates of bacterial proliferation can be assessed by determination of net bacterial load within cells by plating cell or tissue lysates onto fresh laboratory medium cultures and counting of colony-forming units (cfu).

Another method of phenotypic determination involves the observation of infectious progeny or diseased cells for morphological analysis, e.g., by electron microscopy. Quantitative assessment of an infection or disease conducted in the presence of ProTα variants can also be determined using molecular markers, for example, by ELISA assay, reverse transcriptase activity, or DNA synthesis by quantitative PCR using standard techniques known to those skilled in the art.

### Use of cell lines and animal models

A DNA encoding a ProTα variant can be used to engineer cell lines which constitutively express the ProTα variant in order to test the effect of the ProTα variant on different isolates viral, bacterial or fungal strains as well as various types of cancer, ischemia and blood disorders. For testing the efficacy of ProT variants on different viral isolates, such as HIV, cell lines can include lymphotropic and macrophage-tropic strains, primary strains derived from blood cells or tissues, and North American, European, African and Asian isolates. Such methods can allow the selection of a ProTα variant which has optimal inhibitory effect on a particular viral isolate of interest. One of skill in the art will also appreciate that such cell lines can also be used to test the effect of ProTα variants on different viruses (e.g., other retroviruses, lentiviruses, and hepatitis virus) and isolates and strains thereof.

ProTα variants of the invention can be tested in animal models of viral, bacterial or fungal infections. Exemplary animal models of a viral infection include HIV infected animal models, such as the SCID-Hu mouse model of HIV-1 infection (Aldrovandi et al., J. Virol. 70:1505, 1996) and SIV-infected monkeys. Such models of infection are suitable for testing ProTα variants of the invention for efficacy against challenge with HIV or other lentiviruses and other retroviruses in order to identify those ProTα variants which can be used for prevention or treatment of viral infection. Animal models designed for other viral, bacterial or fungal infections may also be utilized in a similar manner by those of skill in the art.

### Experiments to monitor the effective concentration of ProTα variants

ProTα variants can be assayed to determine the concentration required to achieve elimination or reduction in growth of a target infection or disease. A convenient variable for measurement is the concentration of a ProTα variant required to inhibit 50% of pathogen replication or tumor growth (IC₅₀), whether assayed in cell culture or with the use of a molecular marker. An exemplary assay for viral growth includes the measurement of viral p24 production by ELISA assay, presence of viral RNA, reverse transcriptase activity, or viral DNA synthesis by quantitative PCR using standard techniques known to those skilled in the art for the detection of HIV virion levels.

### Monitoring cytotoxicity

ProTα variants of the invention can be evaluated for cytotoxic effects using standard assays that measure cell viability. Such assays include ¹⁴C protein hydrolysate, ³H thymidine uptake, MTT or MTS reduction, and cell growth. Such parameters as TD₅₀ (toxic dose to 50% of the tested culture) can be derived from such assays. Comparison of the TD₅₀ so derived with the IC₅₀ (ProTα variant concentration required to inhibit 50% of the marker for infection or disease being tested) can indicate a therapeutic index for a particular compound (TI). Preferably, the IC₅₀ is at least ten times higher than the TD₅₀, and the IC₅₀ is effective at a minimum of 10⁻⁶ M in a culture with a ProTα variant. Most preferably, a ProTα variant of the invention exhibits an IC₅₀ of 10⁻⁷ M or 10⁻⁸ M.

### ProTα Variants Induce IFNs /RANTES/TNFa for treatment of Viral/Bacterial/Fungal Infections

An "immunological response" or "immune response" is the development in a subject of a humoral and/or a cellular immune response to an antigen or an immunological adjuvant.

As used herein, an "antigen" refers to a molecule containing one or more epitopes (e.g., linear, conformational or both) that elicit an immunological response. The term "antigen" as used herein includes subunit antigens, i.e., antigens which are separate and discrete from a whole organism with which the antigen is associated in nature. Antibodies such as anti-idiotype antibodies, or fragments thereof, and synthetic peptide mimotopes, which can mimic an antigen or antigenic determinant, are also captured under the definition of antigen as used herein.

Cells that play a role in the immune response are immune cells of hematopoietic origin. Immune cells include lymphocytes, such as B cells and T cells; natural killer cells; and myeloid cells, such as monocytes, macrophages, eosinophils, mast cells, basophils, and granulocytes.

As used herein, the term "T cell" includes CD4+ T cells and CD8+ T cells. The term T cell also includes both T helper I type T cells and T helper 2 type T cells.

For purposes of the present invention, a "cellular immune response" or "cell mediated immune response" is one mediated by T-lymphocytes and/or other white blood cells, while a humoral immune response refers to an immune response mediated by antibody molecules. One important aspect of cellular immunity involves an antigen-specific response by cytolytic T-cells (CTLs). CTLs have specificity for peptide antigens that are presented in association with proteins encoded by the major histocompatibility complex (MHC) and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" or "cell mediated immune response" also refers to the production of cytokines, chemokines and other such molecules produced by activated T-cells and/or other white blood cells, including those derived from CD4⁺ and CD8⁺ T-cells.

A composition such as an immunogenic composition or a vaccine that elicits a cellular immune response may thus serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host. The ability of a particular antigen or composition to stimulate a cell-mediated immunological response may be determined by a number of assays known in the art, such as by lymphoproliferation (lymphocyte activation) assays, CTL cytotoxic cell assays, by assaying for T-lymphocytes specific for the antigen in a sensitized subject, or by measurement of cytokine production by T cells in response to restimulation with antigen. Such assays are well known in the art. See, e.g., Erickson et al. (1993) J. Immunol 151:4189-4199; Doe et al. (1994) Eur. J. Immunol 24:2369-2376. Thus, an immunological response as used herein may be one which stimulates the production of CTLs and/or the production or activation of helper T-cells. The antigen of interest may also elicit an antibody-mediated immune response. Hence, an immunological response may include, for example, one or more of the following effects among others: the production of antibodies by, for example, B-cells; and/or the activation of suppressor T-cells and/or γ6 T-cells directed specifically to an antigen or antigens present in the composition or vaccine of interest. These responses may serve, for example, to neutralize infectivity, and/or mediate antibody-complement, or antibody dependent cell cytotoxicity (ADCC) to provide protection to an immunized host. Such responses can be determined using standard immunoassays and neutralization assays, well known in the art.

The term "antibody" refers to an immunoglobulin or antigen-binding fragment thereof, and encompasses any such polypeptide comprising an antigen-binding fragment of an antibody. The term includes but is not limited to polyclonal, monoclonal, monospecific, polyspecific, humanized, human, single-chain, single-domain, chimeric, synthetic, recombinant, hybrid, mutated, grafted, and *in vitro* generated antibodies.

### Inducing IFNs

Interferons (IFNs) are a family of small molecule proteins or glycoproteins produced by eukaryotic cells in response to viral infection and other antigenic stimuli. Interferons can also combat bacterial and parasitic infections and promote a wide range of activities, including inhibition of cell growth, differentiation, and spontaneous apoptosis, and modulation of the immune system, including promotion of antigen presentation, induction of cytokine production, and promotion of T cell maturation and clonal propagation. IFNs represent a generic group of cytokines and are classified into three groups: type I IFNs, type II IFNs and type III IFNs.

The term "interferon type I," "IFN type I," or equivalent denominations, refers to the proteins belonging to the family of type I interferons, of which there are five classes: IFN-alpha (IFN α), IFN-beta (IFN β), IFN-epsilon (IFN-ε), IFN-kappa (IFN-κ) and IFN-omega (IFN-ω). The different human IFN subtypes have been identified by analysis of human cDNA libraries and by protein analysis of the IFNs produced by stimulated lymphoblastoid cells; the reasons for their heterogeneity remain unclear. The function of these cytokines is important in the immune response against multiple types of viral infections, as they initiate mechanisms promoting the apoptosis-induced death of the infected cells and inhibition of viral replication by enhancing antigen presentation.

IFN type I represent useful agents in the immunotherapy of cancer and diseases caused by infectious pathogens, like virus, bacteria, fungi, and parasites, since said proteins can selectively activate the process of destroying only those epithelial cells that are infected with pathogens or are tumoral. IFN type I increases the antigenicity of infected or tumoral epithelial cells, making these cells process and present more antigens at their surface, and therefore becoming more visible and reactive to immune responses, resulting finally into a selective destruction of the same cells.

Interferon α (IFNα) is produced predominantly by B lymphocytes but also by macrophages and can be sub-divided into 13 sub-types. The level of homology between IFNα isotypes is high being around 75-80% identity at the amino acid level. IFNα stimulates the activity of macrophages and Natural Killer (NK) cells to elicit an anti-viral response or an anti-tumor response.

The class of interferons, IFN α subtype 2 (IFN α2), exists in several forms including: IFN α2a and IFN α2b. The two forms are nearly identical except for one amino acid difference at position 23, in which IFN α2a encodes a lysine at said amino acid and IFN α2b encodes arginine. Both IFN α2 forms comprise an anti-viral therapeutic for the treatment of infection by a virus, such as HCV. Due to the instability of the native IFN α2 polypeptides, they are generally administered in a modified or derivatized form, for example pegylated polypeptides.

As used herein, the term "pegylated IFN-α2a" refers to the linkage of interferon (IFN) alpha subtype 2a to polyethylene-glycol (PEG). Pegylated interferon- α2a has a very restricted volume of distribution, longer half-life and reduced clearance compared with native interferon-alpha-2a, and can be given once weekly independently of bodyweight. The slow clearance of pegylated IFN-α2a maintains inhibition of viral replication for a longer time, which leads to more effective treatments and fewer patient administrations.

Type II interferon includes one member, interferon γ (-gamma) and is involved in the regulation of immune and inflammatory responses. In humans interferon γ (IFNγ) is encoded by a single gene and is produced by activated T-cells and NK cells. IFNγ does have anti-viral and anti-tumor activity however this is generally weaker when compared to IFNα. The function of IFNγ is to enhance the effects of Type I interferon by recruiting leukocytes to a site of infection and by stimulating macrophages to engulf invading bacteria during an infection.

The more recently discovered type III IFNs includes three closely related interferon-gamma (IFN-λ) proteins referred to as IFN-λ1, IFN-λ2 and IFN-λ3 also known as IL29, IL28A and IL28B, respectively. Type III interferons have homology to type I interferons and IL10 and similar biological activity; although, they do not inhibit division of some B cell lines. IFN-λ1, IFN-λ2 and IFN-λ3 bind a third receptor distinct from those of type I or type II interferons. Thus they are now termed the type III interferons. These interferons have been shown to have anti-viral activity in *in vitro* cell studies.

### Inducing RANTES

As used herein, the term "RANTES" or "Regulated upon Activation, Normal T-cell Expressed, and Secreted, CCL5" is a protein belonging to the family of chemokines. RANTES is primarily involved in the migration of type 1 T cells and the activation of cytotoxic T cells, and plays an important role in the infection resistance and antitumor resistance of individuals. RANTES is known to induce chemotaxis *in vitro* in monocytes, macrophages, T cells, NK cells, eosinophils and so forth. Inducing the level and activity of RANTES has therapeutic effects against diseases such as infections and tumors in which the type 1 T cell reaction plays a primary role in their defense.

In certain embodiments, RANTES elicits a natural HIV-suppressive factor that is secreted by activated CD8+ T cells and other immune cells. In other embodiments, elevated levels of RANTES are useful for the treatment or prevention of decreased infection resistance to opportunistic infections occurring in burn patients, AIDS patients, cancer patients, encephalitis patients, individuals having suffered serious injuries or undergone major surgery, or individuals subject to stress.

### ProTα Variants Use For Treatment/Prevention

Inhibitors of the invention, such as ProTα variants and/or functional derivatives and fragments thereof, and compositions comprising these inhibitors can be administered for preventative and/or therapeutic treatments. In preventative applications, inhibitors or compositions thereof are administered to a patient before potential exposure to an infectious disease so as to partially or completely prevent transmission of the infectious disease. In a particular aspect of the invention, an infectious disease is caused by a virus (such as, e.g., a retrovirus or lentivirus), and inhibitors or compositions thereof are administered to a patient before potential exposure to the virus so as to partially or completely prevent viral infection. In general, prevention of viral infection involves partially or completely inhibiting essential stages of the viral life cycle (e.g., transcription of essential viral genes or viral replication).

The terms "prevent," "treating," or "treatment", as used herein, include alleviating, abating or ameliorating disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. The terms "treat," "treating" or "treatment", include, but are not limited to, prophylactic and/or therapeutic treatments.

An immune response is potentiated when the subject has a greater capacity to elicit an immune response than the immune response elicited in the absence of the ProTα variants. For example, an immune response to the pathogen is potentiated when the subject has a greater capacity to elicit an immune response to the pathogen than the immune response elicited by substantially equivalent amount of the pathogen in the absence of the ProTα variants. The pathogen may be, for example, a viral pathogen, a bacterial pathogen, or a fungal pathogen. The pathogen may also include bacterially-infected cells, parasitically-infected cells, virally-infected cells. Likewise, an immune response to cancer is potentiated when the subject has a greater capacity to elicit an immune response to the cancer than in the absence of the ProTα variants. Also, an immune response to ischemia is potentiated when the subject has a greater capacity to elicit an immune response to the ischemia than the immune response elicited in the absence of the ProTα variants.

In certain embodiments, the inhibitors of the invention described herein comprise nucleic acid molecules that encode polypeptides useful for the treatment of viral infections, bacterial infections, fungal infections, parasite inventions, or a combination thereof.

In a particular embodiment of the invention, an inhibitor of a viral infection (e.g., an HIV infection) comprises a peptide or polypeptide having an amino acid sequence that substantially corresponds to the novel ProTα variants described herein (*i.e.,* pProTα, IsoA, IsoB, P7-ProTα, Pseudo4a, Pseudo4, oPTMA, ePTMA, kPTMA). An inhibitor of a viral infection (e.g., an HIV infection) may also be a peptide or polypeptide having an amino acid sequence comprising any one of the novel ProTα variants described herein (*i.e.,* pProTα, IsoA, IsoB, P7-ProTα, Pseudo4a, Pseudo4, oPTMA, ePTMA, kPTMA). Such sequences also include orthologs, homologs, derivatives, and functional fragments of any one of the novel ProTα variants described herein.

In one embodiment of the invention the inhibitors of the invention may be used in compositions for the prevention or treatment of an HIV or other lentiviral and retroviral infection, and the treatment of consequent pathologic conditions such as AIDS. Another aspect of the invention, therefore, is directed to methods for preventing and treating an HIV or other lentiviral or retroviral infection by administering a composition containing one or more of the inhibitors the invention to an individual infected with or exposed to HIV for a time and under conditions to accomplish such result.

The novel ProTα variants, compositions and methods of the invention can be used in the treatment of HIV-positive individuals, including those exhibiting the conditions of AIDS-related complex (ARC) and AIDS, as well as those who are asymptomatic. These inhibitors, compositions and methods can also be used in the prophylaxis of HIV or other lentiviral and retroviral infections, and can also be used the treatment or prophylaxis of veterinary infections caused by lentiviruses and other retroviruses.

ProTα variant polypeptides of the present invention can be used to treat infections caused by a virus including, but not limited to, human immunodeficiency virus (HIV), hepatitis B virus (HBV), hepatitis C virus (HCV), Hepatatis A Virus, Influenza, Varicella-Zoster Virus, adenovirus, parvovorus, norovirus, human papilloma virus (HPV), rhinovirus, yellow fever virus, rabies virus, Orthomyxoviruses, such as Influenza A, B and C; Paramyxoviridae viruses, such as Pneumoviruses (RSV), Paramyxoviruses (PIV), Metapneumovirus and Morbilliviruses (e.g., measles); Pneumoviruses, such as Respiratory syncytial virus (RSV), Bovine respiratory syncytial virus, Pneumonia virus of mice, and Turkey rhinotracheitis virus; Paramyxoviruses, such as Parainfluenza virus types 1 - 4 (PIV), Mumps, Sendai viruses, Simian virus 5, Bovine parainfluenza virus, Nipahvirus, Henipavirus and Newcastle disease virus; Poxviridae, including a Orthopoxvirus such as *Variola vera* (including but not limited to, *Variola major* and *Variola minor*); Metapneumoviruses, such as human metapneumovirus (hMPV) and avian metapneumoviruses (aMPV); Morbilliviruses, such as Measles; Picornaviruses, such as Enteroviruses, Rhinoviruses, Heparnavirus, Parechovirus, Cardioviruses and Aphthoviruses; Enteroviruseses, such as Poliovirus types 1, 2 or 3, Coxsackie A virus types 1 to 22 and 24, Coxsackie B virus types 1 to 6, Echovirus (ECHO) virus types 1 to 9, 11 to 27 and 29 to 34 and Enterovirus 68 to 71, Bunyaviruses, including a Orthobunyavirus such as California encephalitis virus; a Phlebovirus, such as Rift Valley Fever virus; a Nairovirus, such as Crimean-Congo hemorrhagic fever virus; Togaviruses (Rubella), such as a Rubivirus, an Alphavirus, or an Arterivirus; Flaviviruses, such as Tick-borne encephalitis (TBE) virus, Dengue (types 1, 2, 3 or 4) virus, Yellow Fever virus, Japanese encephalitis virus, Kyasanur Forest Virus, West Nile encephalitis virus, St. Louis encephalitis virus, Russian spring-summer encephalitis virus, Powassan encephalitis virus; Pestiviruses, such as Bovine viral diarrhea (BVDV), Classical swine fever (CSFV) or Border disease (BDV); Hepadnaviruses, such as Hepatitis B virus, Hepatitis C virus; Rhabdoviruses, such as a Lyssavirus (Rabies virus) and Vesiculovirus (VSV), Caliciviridae, such as Norwalk virus, and Norwalk-like Viruses, such as Hawaii Virus and Snow Mountain Virus; Coronaviruses, such as SARS, Human respiratory coronavirus, Avian infectious bronchitis (IBV), Mouse hepatitis virus (MHV), and Porcine transmissible gastroenteritis virus (TGEV); Retroviruses such as an Oncovirus, a Lentivirus or a Spumavirus; Reoviruses, as an Orthoreovirus, a Rotavirus, an Orbivirus, or a Coltivirus; Parvoviruses, such as Parvovirus B19; Delta hepatitis virus (HDV); Hepatitis E virus (HEV); Hepatitis G virus (HGV); Human Herpesviruses, such as, by way Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8); Papovaviruses, such as Papillomaviruses and Polyomaviruses, Adenoviruess and Arenaviruses, and the like.

ProTα variant polypeptides of the present invention can be used to treat infections caused by a bacterium including, but not limited to, Neisseria meningitides, Streptococcus pneumoniae, Streptococcus pyogenes, Moraxella catarrhalis, Bordetella pertussis, Burkholderia sp. (e.g., Burkholderia mallei, Burkholderia pseudomallei and Burkholderia cepacia), Staphylococcus aureus, Staphylococcus epidermis, Haemophilus influenzae, *Clostridium tetani* (Tetanus), *Clostridium perfringens*, *Clostridium botulinums* (Botulism), *Cornynebacterium diphtheriae* (Diphtheria), *Pseudomonas aeruginosa, Legionella pneumophila, Coxiella burnetii, Brucella* sp. (e.g., *B. abortus, B. canis, B. melitensis, B. neotomae, B. ovis, B. suis and B. pinnipediae,), Francisella sp. (e.g., F. novicida, F. philomiragia* and *F. tularensis), Streptococcus agalactiae, Neiserria gonorrhoeae, Chlamydia trachomatis, Treponema pallidum* (Syphilis), *Haemophilus ducreyi, Enterococcus faecalis, Enterococcusfaecium, Helicobacter pylori, Staphylococcus saprophyticus, Yersinia enterocolitica, E. coli* (such as enterotoxigenic *E. coli* (ETEC), enteroaggregative *E. coli* (EAggEC), diffusely adhering *E. coli* (DAEC), enteropathogenic *E*. *coli* (EPEC), extraintestinal pathogenic *E. coli* (ExPEC; such as uropathogenic *E.coli* (UPEC) and meningitis/sepsis-associated *E.coli* (MNEC)), and/or enterohemorrhagic *E. coli* (EHEC), *Bacillus anthracis* (anthrax), *Yersinia pestis* (plague), *Mycobacterium tuberculosis, Rickettsia, Listeria monocytogenes, Chlamydia pneumoniae, Vibrio cholerae, Salmonella typhi* (typhoid fever), *Borrelia burgdorfer, Porphyromonas gingivalis, Klebsiella, Mycoplasma pneumoniae,* and the like.

ProTα variant polypeptides of the present invention can be used to treat infections caused by a fungus including, but not limited to, Dermatophytres including: *Epidermophytonfloccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum*, *Trichophyton schoenleini, Trichophyton tonsurans, Trichophyton verrucosum, T. verrucosum* var. album, var. discoides, var. ochraceum, *Trichophyton violaceum,* and/or *Trichophyton faviforme;* or from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Microsporidia, Encephalitozoon* spp., *Septata intestinalis* and *Enterocytozoon bieneusi;* the less common are *Brachiola* spp, *Microsporidium* spp., *Nosema* spp., *Pleistophora* spp., *Trachipleistophora* spp., *Vittaforma* spp *Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.*

ProTα variant polypeptides of the present invention can be used to treat infections caused by a parasitic infections including, but not limited to, protozoa, amoeba, sporozoa, Plasmodium spp.(e.g., falciparum, malariae, ovate, and vivax), Plasmodium spp.; Babesia microti; Babesia divergens; Leishmania tropica; Leishmania spp.; Leishmania braziliensis; Leishmania donovani; Trypanosoma gambiense; Trypanosoma rhodesiense; Trypanosoma cruzi (Chagas' disease); Toxoplasma gondii; Cryptosporidium spp.; Toxoplasma gondii; and Sarcocystis spp.; Theileria spp.; and Eimeria spp.

The invention also provides methods of treating, preventing, alleviating a symptom of, or otherwise mitigating cancer or a clinical indication associated with cancer in a subject. The invention is based on the discovery that expression or activity of ProTα variants could inhibit cancerous growth alone or when administered as an adjuvant for any vaccine.

ProTα variant polypeptides of the present invention can be used to treat cancer including, but not limited to the following cancers: breast, ovary, cervix, prostate, testis, genitourinary tract, esophagus, larynx, glioblastoma, neuroblastoma, stomach, skin, keratoacanthoma, lung, epidermoid carcinoma, large cell carcinoma, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, Hodgkin's, hairy cells, buccal cavity and pharynx (oral), lip, tongue, mouth, pharynx, small intestine, colon-rectum, large intestine, rectum, brain and central nervous system, and leukemia.

The invention also provides methods of treating, preventing, alleviating a symptom of, or otherwise mitigating myeloproliferative diseases such as chronic blood disorders including, polycythemia vera (PV) myelofibrosis and essential thrombocythemia (ET). Other myeloproliferative diseases which can be treated by administration of ProTα variant polypeptides include, but are not limited to: thrombocythemia, agnoneic myeloid metaplasia (AMM), also referred to as idiopathic myelofibrosis (IMF), and chronic myelogenous leukemia (CML). The ProTα variant polypeptides of the invention can be used to alleviate the clinical progression of vascular complications resulting from myeloproliferative disease including stroke, cardiovascular events, deep vein thrombosis and pulmonary embolism.

The invention also provides methods of treating, preventing, alleviating a symptom of, or otherwise mitigating ischemia, a clinical indication associated with ischemia and/ or reperfusion injury in a subject. The invention is based on the discovery that expression or activity of ProTα variants inhibit ischemia and/or reperfusion injury by reduction of necrosis and apoptosis. Accordingly, the invention provides methods of preventing or inhibiting ischemia, a clinical indication associated with ischemia, reperfusion injury, in a subject, in a bodily tissue and/or in a tissue or organ to be transplanted.

The ischemia to be treated using ProTα variants of the invention includes, but is not limited to, cardiac ischemia, cerebral ischemia, renal ischemia, and related ischemic diseases or events. The heart, the kidneys, and the brain are among the organs that are the most sensitive to inadequate blood supply. Ischemia in brain tissue is due, for example, to stroke or head injury. Clinical indications associated with ischemia and reperfusion include, for example, coronary artery disease, cerebral vascular disease, cardiac ischemia, myocardial ischemia, renal ischemia and peripheral vascular disease. Ischemia is a feature of heart diseases including atherosclerosis, myocardial infarction, transient ischemic attacks, cerebrovascular accidents, ruptured arteriovenous malformations, and peripheral artery occlusive disease. For example, a ProTα variant of the invention is administered to a subject in need thereof before an ischemic event, during an ischemic event, after an ischemic event or any combination thereof.

In another aspect of the invention, the inhibitors of the invention are a pharmaceutical composition suitable for administration to a mammal, preferably a human. To administer the inhibitors composition to humans or animals, it is preferable to formulate the molecules in a composition comprising one or more pharmaceutically acceptable carriers. The phrase "pharmaceutically or pharmacologically acceptable" refers to molecular entities and compositions that do not produce allergic, or other adverse reactions when administered using routes well-known in the art. "Pharmaceutically acceptable carriers" include any and all clinically useful solvents, dispersion media, coatings, antibacterial and antifungal agents, isotonic and absorption delaying agents and the like.

Examples of pharmaceutically acceptable carriers or additives include water, a pharmaceutical acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, glycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the like. Additives used are chosen from, but not limited to, the above or combinations thereof, as appropriate, depending on the dosage form of the present invention.

The inhibitors of the invention may be used alone or in combination with other known or to be discovered inhibitors of HIV replication, including, but not limited to, other antiviral compounds, immunomodulators, antibiotics, vaccines, chemokines and other therapeutic agents. Particular agents which can be used in combination with the inhibitors of the invention include, but are not limited to, azidothymidine (AZT), dideoxyinosine (DDI), dideoxycytosine (DDC), saquinavir, indinavir, ritonavir, and other antiviral compounds. The inhibitors of the invention may also be used in combination with agents which are used to treat secondary complications of HIV infection, e.g., gancyclovir used in the treatment of cytomegalovirus retinitis. Combination therapy may retard the development of drug-resistant mutants by requiring multiple mutation events for the emergence of a fully drug-resistant isolate.

Other examples of agents the inhibitors of this invention may also be combined with include, without limitation, The inhibitors of the invention may be used alone or in combination with other known or to be discovered inhibitors of HIV replication, including, but not limited to, other antiviral compounds, immunomodulators, antibiotics, vaccines, chemokines and other therapeutic agents. Particular agents which can be used in combination with the inhibitors of the invention include, but are not limited to, azidothymidine (AZT), dideoxyinosine (DDI), dideoxycytosine (DDC), saquinavir, indinavir, ritonavir, and other antiviral compounds. The inhibitors of the invention may also be used in combination with agents which are used to treat secondary complications of HIV infection, e.g., gancyclovir used in the treatment of cytomegalovirus retinitis. Combination therapy may retard the development of drug-resistant mutants by requiring multiple mutation events for the emergence of a fully drug-resistant isolate.

The inhibitors of the present invention may be administered to a host as a composition in an amount effective to inhibit HIV infection and/or transcription/replication in target cells. The compositions contain an effective dosage of at least one of the inhibitors of the present invention, together with an acceptable carrier.

In that pProTα is a remarkably stable protein at low pH (pH of CVL is ∼4), it is well suited for pharmaceutical applications and as a microbicide candidate.

The inhibitors of the invention may be systematically administered for preventing and/or treating an HIV or other lentiviral or retroviral infection. When used systemically, the inhibitor compositions may be formulated as liquids, pills, tablets, lozenges or the like, for enteral administration, or in liquid form for parenteral injection. The peptides and/or polypeptides (or inhibitor-protein conjugates) may be combined with other ingredients such as carriers and/or adjuvants. There are no limitations on the nature of such other ingredients, except that they must be physiologically acceptable, efficacious for their intended administration and cannot degrade the activity of the active ingredients of the compositions. A ProTα variant can also be covalently attached to a protein carrier, such as albumin, so as to minimize diffusion of the inhibitor.

The compositions described herein may be administered in combination with a second therapeutic agent. For example, for cancer treatment, a chemotherapeutic agent may be used as the second agent. For treatment of autoimmune diseases, non-steroidal anti-inflammatory drugs (NSAIDs), analgesiscs, glucocorticoids, disease-modifying antirheumatic drugs (DMARDs), may be used as the second agent.

Cancer therapies include, but are not limited to, surgery and surgical treatments, radiation therapy, and therapeutic agents (*e.g.,* biotherapeutic agents and chemotherapeutic agents).

Exemplary biotherapeutic agents include, but are not limited to, interferons, cytokines (*e.g.,* tumor necrosis factor, interferon α, interferon γ), vaccines, hematopoietic growth factors, monoclonal serotherapy, immunostimulants and/or immunodulatory agents (e.g., IL-1, 2, 4, 6, or 12), immune cell growth factors (*e.g.,* GM-CSF) and antibodies (*e.g.* HERCEPTIN (trastuzumab), T-DM1, AVASTIN (bevacizumab), ERBITUX (cetuximab), VECTIBIX (panitumumab), RITUXAN (rituximab), BEXXAR (tositumomab)).

Exemplary chemotherapeutic agents include, but are not limited to, antiestrogens *(e.g.* tamoxifen, raloxifene, and megestrol), LHRH agonists *(e.g.* goscrclin and leuprolide), anti-androgens *(e.g.* flutamide and bicalutamide), photodynamic therapies *(e.g.* vertoporfin (BPD-MA), phthalocyanine, photosensitizer Pc4, and demethoxy-hypocrellin A (2BA-2-DMHA)), nitrogen mustards (*e.g.* cyclophosphamide, ifosfamide, trofosfamide, chlorambucil, estramustine, and melphalan), nitrosoureas (*e.g.* carmustine (BCNU) and lomustine (CCNU)), alkylsulphonates *(e.g.* busulfan and treosulfan), triazenes *(e.g.* dacarbazine, temozolomide), platinum containing compounds (e.g. cisplatin, carboplatin, oxaliplatin), vinca alkaloids (*e.g.* vincristine, vinblastine, vindesine, and vinorelbine), taxoids (*e.g*. paclitaxel or a paclitaxel equivalent such as nanoparticle albumin-bound paclitaxel (ABRAXANE), docosahexaenoic acid bound-paclitaxel (DHA-paclitaxel, Taxoprexin), polyglutamate bound-paclitaxel (PG-paclitaxel, paclitaxel poliglumex, CT-2103, XYOTAX), the tumor-activated prodrug (TAP) ANG1005 (Angiopep-2 bound to three molecules of paclitaxel), paclitaxel-EC-1 (paclitaxel bound to the erbB2-recognizing peptide EC-1), and glucose-conjugated paclitaxel, *e.g.,* 2'-paclitaxel methyl 2-glucopyranosyl succinate; docetaxel, taxol), epipodophyllins (*e.g.* etoposide, etoposide phosphate, teniposide, topotecan, 9-aminocamptothecin, camptoirinotecan, irinotecan, crisnatol, mytomycin C), antimetabolites, DHFR inhibitors (*e.g.* methotrexate, dichloromethotrexate, trimetrexate, edatrexate), IMP dehydrogenase inhibitors (e.g. mycophenolic acid, tiazofurin, ribavirin, and EICAR), ribonuclotide reductase inhibitors (e.g. hydroxyurea and deferoxamine), uracil analogs (e.g. 5-fluorouracil (5-FU), floxuridine, doxifluridine, ratitrexed, tegafur-uracil, capecitabine), cytosine analogs (e.g. cytarabine (ara C), cytosine arabinoside, and fludarabine), purine analogs (e.g. mercaptopurine and Thioguanine), Vitamin D3 analogs (e.g. EB 1089, CB 1093, and KH 1060), isoprenylation inhibitors (*e.g.* lovastatin), dopaminergic neurotoxins *(e.g.* 1-methyl-4-phenylpyridinium ion), cell cycle inhibitors *(e.g.* staurosporine), actinomycin *(e.g.* actinomycin D, dactinomycin), bleomycin *(e.g.* bleomycin A2, bleomycin B2, peplomycin), anthracycline (*e.g.* daunorubicin, doxorubicin, pegylated liposomal doxorubicin, idarubicin, epirubicin, pirarubicin, zorubicin, mitoxantrone), MDR inhibitors (*e.g.* verapamil), Ca²⁺ ATPase inhibitors (e.g. thapsigargin), imatinib, thalidomide, lenalidomide, tyrosine kinase inhibitors (*e.g*., axitinib (AG013736), bosutinib (SKI-606), cediranib (RECENTIN™, AZD2171), dasatinib (SPRYCEL®, BMS-354825), erlotinib (TARCEVA®), gefitinib (IRESSA®), imatinib (Gleevec®, CGP57148B, STI-571), lapatinib (TYKERB®, TYVERB®), lestaurtinib (CEP-701), neratinib (HKI-272), nilotinib (TASIGNA®), semaxanib (semaxinib, SU5416), sunitinib (SUTENT®, SU11248), toceranib (PALLADIA®), vandetanib (ZACTIMA®, ZD6474), vatalanib (PTK787, PTK/ZK), trastuzumab (HERCEPTIN®), bevacizumab (AVASTIN®), rituximab (RITUXAN®), cetuximab (ERBITUX®), panitumumab (VECTIBIX®), ranibizumab (Lucentis®), nilotinib (TASIGNA®), sorafenib (NEXAVAR®), everolimus (AFINITOR®), alemtuzumab (CAMPATH®), gemtuzumab ozogamicin (MYLOTARG®), temsirolimus (TORISEL®), ENMD-2076, PCI-32765, AC220, dovitinib lactate (TKI258, CHIR-258), BIBW 2992 (TOVOK™), SGX523, PF-04217903, PF-02341066, PF-299804, BMS-777607, ABT-869, MP470, BIBF 1120 (VARGATEF®), AP24534, JNJ-26483327, MGCD265, DCC-2036, BMS-690154, CEP-11981, tivozanib (AV-951), OSI-930, MM-121, XL-184, XL-647, and/or XL228), proteasome inhibitors (*e.g.,* bortezomib (VELCADE)), mTOR inhibitors *(e.g.,* rapamycin, temsirolimus (CCI-779), everolimus (RAD-001), ridaforolimus, AP23573 (Ariad), AZD8055 (AstraZeneca), BEZ235 (Novartis), BGT226 (Novartis), XL765 (Sanofi Aventis), PF-4691502 (Pfizer), GDC0980 (Genetech), SF1126 (Semafoe) and OSI-027 (OSI)), oblimersen, gemcitabine, carminomycin, leucovorin, pemetrexed, cyclophosphamide, dacarbazine, procarbizine, prednisolone, dexamethasone, campathecin, plicamycin, asparaginase, aminopterin, methopterin, porfiromycin, melphalan, leurosidine, leurosine, chlorambucil, trabectedin, procarbazine, discodermolide, carminomycin" aminopterin, and hexamethyl melamine.

As used herein, a physiologically acceptable carrier includes any and all solvents, dispersion media, coatings, antibacterial and antifungal agents, isotonic agents and the like. The use of such media and agents are well-known in the art.

The forms of the compositions suitable for injection include sterile aqueous solutions or dispersions and sterile powders for the extemporaneous preparation of sterile injectable solutions or dispersions. In all cases the ultimate solution form must be sterile and fluid. Typical carriers include a solvent or dispersion medium containing, for example, water buffered aqueous solutions (i.e., biocompatible buffers), ethanol, polyol such as glycerol, propylene glycol, polyethylene glycol, suitable mixtures thereof, surfactants or vegetable oils. Sterilization can be accomplished by an art-recognized technique, including but not limited to, filtration or addition of antibacterial or antifungal agents, for example, paraben, chlorobutanol, phenol, sorbic acid or thimerosal. Further, isotonic agents such as sugars or sodium chloride may be incorporated in the subject compositions.

Production of sterile injectable solutions containing the subject inhibitors is accomplished by incorporating these compounds in the required amount in the appropriate solvent with various ingredients enumerated above, as required, followed by sterilization, preferably filter sterilization. To obtain a sterile powder, the above solutions are vacuum-dried or freeze-dried as necessary.

The polypeptide compositions also may be impregnated into transdermal patches, plasters and bandages, preferably in a liquid or semi-liquid form.

When the inhibitors of the invention are administered orally, the compositions thereof containing an effective dosage of the peptide may also contain an inert diluent, an assimilable edible carrier and the like, be in hard or soft shell gelatin capsules, be compressed into tablets, or may be in an elixir, suspension, syrup or the like.

As indicated above, the inhibitors may be administered by any convenient route, for example by infusion or bolus injection, by absorption through epithelial or mucocutaneous linings (e.g., oral mucosa, rectal, vaginal, and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a particular embodiment, it may be desirable to administer the pharmaceutical compositions of the invention locally, e.g., by local infusion during surgery, topical application, e.g., by injection, by means of a catheter, or by means of an implant, said implant being of a porous, non-porous, or gelatinous material, including membranes, such as silastic membranes, or fibers.

The subject inhibitors are thus compounded for convenient and effective administration in physiologically effective amounts with a suitable pharmaceutically acceptable carrier in a therapeutically effective dosage. Examples of suitable pharmaceutical carriers are described in "Remington's Pharmaceutical Sciences" by E. W. Martin, incorporated in its entirety by reference herein.

The precise effective amount of inhibitors to be used in the methods of this invention to prevent or treat an HIV infection cannot be stated because of the nature of the infectious process. It must be noted that the amount of ProTα variant to be administered will vary with the degree of infection in an individual, as determined by such parameters as viral load and CD4 cell counts. Individual-specific variables such as age, weight, general health, gender, diet, and intake of other pharmaceuticals can factor into the choice of dosage. The design of an optimal protocol for an infected individual may further consider the identity of the viral isolate(s) isolated from an infected individual with an infection for optimal result. A further consideration in protocol design would be the presence of a viral strain which is already resistant to existing protease or reverse transcriptase inhibitors.

The amount of a ProTα variant of the invention per unit volume of composition for administration depends upon the amount of active ingredients that are afforded directly to the site of infection. However, it can generally be stated that a peptide or polypeptide of the invention should preferably be present in an amount of at least about 1.0 ng/ml of combined composition, more preferably in an amount up to about 1.0 milligram per milliliter.

Systemic dosages depend on the age, weight and condition of the individual and on the administration route. In general, for example, a suitable dosage for the administration to adult humans ranges from about 0.01 to about 100 mg per kilogram body weight. The preferred dosage ranges from about 0.5 to about 5.0 mg per kilogram body weight.

The amount of the compound of the invention which will be effective in the prevention and/or treatment of an infectious disease (e.g., viral infection) can be determined by standard clinical techniques based on the present description. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Suppositories generally contain active ingredient in the range of 0.5% to 10% by weight; oral formulations preferably contain 10% to 95% active ingredient. Effective doses may be extrapolated from dose-response curves derived from in vitro or animal model test systems.

Since the inhibitory compositions of this invention are effective in reducing or eliminating the ability of HIV or other lentiviruses and other retroviruses to generate infectious progeny, periodic readministration of the compositions may be indicated and preferred.

The peptide and polypeptide inhibitors of the invention can also be delivered to an individual by administering a vector that comprises and expresses a nucleic acid encoding the inhibitor. DNAs encoding one or more of the inhibitors of the invention can be delivered to the cells of an individual in need of such an inhibitor by any method of gene transfer known to those skilled in the art, including, but not limited to, viral vectors, lipid-mediated delivery, transfection, electroporation, as well as other methods. Viral vectors which can be used to deliver such inhibitors include those derived from DNA and RNA viruses, including, but not limited to, adenovirus, herpesvirus, poxvirus, retrovirus, and adeno-associated virus.

Parameters, which are used to monitor the effect of a ProTα variant of the invention administered to an individual with an established HIV infection or administered to an individual for prophylaxis, include the use of CD4 counts, plasma viral RNA concentration, viral phenotype, p24 antigen concentration, viral phenotype, level of anti-HIV antibodies as well as other markers of the clinical progression of an HIV infection known to those skilled in the art.

It will be recognized that the inhibitors and methods of the invention can be used in the treatment or prevention of any other lentiviral or retroviral infection, including, but not limited to, those resulting from HIV-1, HIV-2, simian immunodeficiency virus (SIV), feline immunodeficiency virus (FIV), bovine immunodeficiency virus (BIV), visna virus and all strains and isolates thereof.

Kits can also be supplied for use with the ProTα variants of the present invention. Thus, the polypeptides are typically provided in lyophilized form, either alone or in conjunction with buffers, stabilizers, inert proteins, or the like, in accordance with well-known manufacturing procedures. Other pharmaceutically acceptable buffers include: phosphate-buffered saline, Ringer's solution, or dextrose solution. It can also contain other materials useful to the end-user, including other pharmaceutically acceptable formulating solutions such as buffers, diluents, filters, needles, and syringes or other delivery device. The kit may further include an adjuvant (such as an aluminum containing adjuvant or MF59).

In one embodiment, the kit comprises a container containing the ProTα polypeptides and instructions for use. The invention also provides a delivery device prefilled with the immunogenic compositions of the invention.

The practice of the invention employs, unless otherwise indicated, conventional techniques of protein chemistry, molecular virology, microbiology, recombinant DNA technology, and pharmacology, which are within the skill of the art. Such techniques are explained fully in the literature. See, e.g., Current Protocols in Molecular Biology, Ausubel et al., eds., John Wiley & Sons, Inc., New York, 1995; Remington's Pharmaceutical Sciences, 17th ed., Mack Publishing Co., Easton, Pa., 1985; and Molecular Cloning: A Laboratory Manual, Sambrook et al., eds., 2nd edition, Cold Spring Harbor Press, Cold Spring Harbor, N.Y., 1989.

### EXEMPLIFICATION

The invention now being generally described, it will be more readily understood by reference to the following examples, which are included merely for purposes of illustration of certain aspects and embodiments of the present invention, and are not intended to limit the invention.

### Methods and Materials:

### Reagents

Recombinant human c ProTα and antibody to c ProTα were purchased from Alexis Biochemicals (San Diego, CA). IFN-αl-human was purchased from PBL InterferonSource (Piscataway, NJ). Poly I:C was purchased from Sigma-Aldrich.

### Viruses

HIV-1_{BaL} was purchased from ABI (Columbia, Maryland). The R5 virus HIV-1_{BaL}, originally isolated from lung tissue and passed in primary macrophages, can be used to infect primary macrophages, CD4⁺ cells or other cells.

### Isolation of Human Monocyte-Derived Macrophages and CD8⁺ Cells

Macrophages (CD14⁺) and CD8⁺ cells can be prepared by isolating an enriched population of monocytes from peripheral blood mononuclear cells (PBMCs) derived from healthy adult donors by using standard Ficoll-Hypaque purification (Sigma Aldrich). CD14⁺ and CD8⁺ cells can be separated from monocytes by CD 14- and CD8-immunomagnetic bead enrichment, respectively (Miltenyi Biotec Auburn, CA). Next, CD14⁺ cells can be differentiated into macrophages and CD8⁺ cells can be immortalized, as described below.

CD8⁺ cells can be prepared and transformed by herpesvirus saimiri (HVS), as previously described, to generate CD8⁺ K#1 50K cells, which were shown to have a potent anti-HIV-1 activity (Mosoian, A., Teixeira, A., Caron, E., Piwoz, J. and Klotman, M. 2000. CD8+ cell lines isolated from HIV-1-infected children have potent soluble HIV-1 inhibitory activity that differs from beta-chemokines. Viral Immunol. 13(4):481-95). Purity of the cell populations was confirmed by FACS analysis. The cell populations was more than 95% positive for the selected population of cells.

### Cell Culture

CD14⁺ monocytes isolated from healthy adult donors were differentiated into primary macrophages by culture for 10-15 days in Dulbecco's modified Eagle medium (DMEM) containing 10% fetal bovine serum (FBS).

Isolated CD8⁺ cells were cultured for 24 hours in serum-free RPMI supplemented with IL-2 (50 U/mL), insulin (50 ng/mL), and transferrin (Sigma) before the cell-conditioned medium was filtered and supernatant was collected for fractionation.

The ectocervical cells were cultured as described in Fichorova, R.N., Rheinwald, J.G., and Anderson, D.J. 1997. Biol Reprod 57:847-855.

### Isolation of Protein Fractions

To subdivide media supernatant into discreet protein fractions, CD8⁺ cell supernatants were loaded onto a MonoQ (GE Healthcare) column by using an AKTA explorer 100 (GE Healthcare). The column was washed with a buffer containing 50 mM phosphate buffer (pH 7.4) and 100 mM NaCl to remove unbound material. The first fraction of bound molecules was eluted with five column volumes of a buffer containing 50 mM phosphate buffer (pH 7.4) and 200 mM NaCl. The remaining seven fractions were each eluted by sequentially increasing the concentration of NaCl in the phosphate buffer by 100 mM for each step, over a range of 300 to 900 mM, and by using five column volumes of buffer for each fraction. The fractions were dialyzed across 0.5 kDa MWCO dialysis membrane against 50 mM phosphate buffer at pH 7.4. Dialyzed samples were concentrated by lyophylization. Protein concentrations were determined by BCA assay (Pierce). CVL fluid fractions were obtained in the same manner.

### Protein sequencing

To confirm the identities of ProTα variants, peptides were sequenced. Equivalent protein fractions from successive gel filtration chromatography steps were pooled. Aliquots of each pool were digested with trypsin and analyzed by using an LCQ ion trap mass spectrometer (Thermo-Finnigan, San Jose, CA) fitted with a custom-built nanoflow-high-pressure liquid chromatography microelectrospray ionization source (43a). Tandem mass spectrometry spectra were searched against the human non-redundant database (NCBI, Bethesda, MD), de novo sequenced, and manually validated.

### Poly I:C cell treatment

Ectocervical cells were transfected with Poly I:C using Lipofectamine 2000 (Invitrogen) according to manufacturers instructions. Macrophages were treated with Poly I:C without transfection

### RNA Extraction

To assess the expression level of mRNA transcripts, total RNA was extracted from treated cells using RNeasy spin columns (Qiagen), according to the manufacturer's protocol. The total RNA served as a template for the in vitro amplification of mRNA encoding one of the 3 ProTα variants, cProTα, type I IFNs, type III IFNs and RANTES. The concentration of the RNA samples was determined by measuring optical density at 260nm. After treatment with RNase-free DNase (Qiagen), reverse transcription was performed on 1 µg of total RNA to generate cDNA by using Omniscript RT kit (Qiagen) and oligo dT primers, according to the manufacturer's protocol. The concentration of the cDNA samples was determined by measuring the optical density at 260nm.

### Quantitative RT-PCR

For amplification of mRNA transcripts, RT-qPCR was performed on an ABI Prism 7700. Reactions were carried out in 10 µL by using SYBR Green PCR master mix according to the manufacturer's protocol (Qiagen) with 5-10 ng of cDNA. The human ribosomal protein S11 was amplified in parallel with ProTα or cytokine products and used as a reference housekeeping gene in each RT-PCT experiment. The concentration of primer pairs was 300 nM. All samples and controls were performed in triplicate.

### Primers for FIGS 1A-C:

Human cProTα
Forward-ATG TCA GAC GCA GCC GTA GAC (SEQ ID NO: 19)
Reverse-CGT TAG CAG GGG CGT CTC TTC (SEQ ID NO: 20)

### Human pProTα- CVL

### F-full-CVL as cProTa

R-112CVL-PTMA CTTCCTCCTCCTCTTCCTCCCCAC (SEQ ID NO: 21)
F-219CVL-PTMA GTGGGGAGGAAGAGGAGGAGGAAG (SEQ ID NO: 22)
R-CVL-Full-PTMA CTAGTCATCCTCGTCGGTCTTCTGAC (SEQ ID NO: 23)

### Human iso-A ProTα

IsoA-5UTR-F AATGTTCATGTGCCCATCGCAGTC (SEQ ID NO: 24)
IsoA-3UTR-R GAGGCGGATGAGGAACAATGCAAA (SEQ ID NO: 25)

### Human iso-B ProTα

F-full-isoB and cPTMA- ATG TCA GAC GCA GCC GTA GAC (SEQ ID NO: 26)
F-isoB128bp- GCTAACGGGAATGCTGTGAGGAAGAG (SEQ ID NO: 27)
R-isoB134bp- CTCTTCCTCACAGCATTCCCGTTAGC (SEQ ID NO: 28)
R-isoB-full- TTTAACTTTTCCTTTTTTGCTGTC (SEQ ID NO: 29)

### Human breast cancer-derived ProTα

### F-full-same as cProTa

R-187-BC CTTCCTCCCCACCTTCTTCCTC (SEQ ID NO: 30)
F-131-BC GAGGAAGAAGGTGGGGAGGAAG (SEQ ID NO: 31) R-full BC same as cProTa

### P7 B ProTα to 5' and 3' un-translated region

P7-F TCATCCGCCTCCTTGCTCG (SEQ ID NO: 32)
P7-R ATTCTCATGGTTTGGGTTGGGTGG (SEQ ID NO: 33)

### Human RPS11

Forward-GCCGAGACTATCTGCACTAC (SEQ ID NO: 34)
Reverse -ATGTCCAGCCTCAGAACTTC (SEQ ID NO: 35)

### Primers for FIG 3:

### Human IFN-β

Forward-GTCAGAGTGGAAATCCTAAG (SEQ ID NO: 36)
Reverse-ACAGCATCTGCTGGTTGAAG (SEQ ID NO: 37)

### Human type III IFN

### (lambda 2)

IL-28A-F ACAGCCTCAGAGTGTTTCTTCTGC (SEQ ID NO: 38)
IL28A-R ACAGGGACTTGAACTGGGCTATGT (SEQ ID NO: 39) (lambda 1)
h-IL-29(L1)-F ACCTCAAATATGTGGCCGATGGGA (SEQ ID NO: 40)
h-IL-29(L1)-R AAGGAGTAGGGCTCAGCGCATAAA (SEQ ID NO: 41)

### Human type I IFNa1

Forward-CTGAATGACTTGGAAGCCTG (SEQ ID NO: 42)
Reverse-ATTTCTGCTCTGACAACCTC (SEQ ID NO: 43)

### Human RANTES

Forward-TCCAACCCAGCAGTCGTCT (SEQ ID NO: 44)
Reverse-TTGGCGGTTCTTTCGGGTG (SEQ ID NO: 45)

### Mouse specific IFN-β primers

Forward-AGATGTCCTCAACTGCTCTC (SEQ ID NO: 46)
Reverse-AGATTCACTACCAGTCCCAG (SEQ ID NO: 47)

### Mouse RPS11

Forward-CGTGACGAAGATGAAGATGC (SEQ ID NO: 48)
Reverse -GCACATTGAATCGCACAGTC (SEQ ID NO: 49)

### Primers for FIG 4:

### Human IFNγ

Forward AGGCAAGGCTATGTGATTAC (SEQ ID NO: 50)
Reverse GCTATGTTTTCATCAGGGTC (SEQ ID NO: 51)

### Relative Quantification of PCR transcripts using CT method

To make comparisons between samples and controls, the CT (cycle threshold, defined as the cycle number at which the fluorescence is above the fixed threshold) values obtained for the specified target genes were normalized to the CT of ribosomal protein cDNA in each sample. RT-PCR products were separated by size using denaturing gel electrophoresis.

### MTS Cell Viability Assay

To assess the effects of ProTα variants on cell viability, an MTS cell proliferation assay was performed according to manufacturer's instructions (Promega). Macrophages were treated with different variants of ProTα (3ng/ml) or as a positive control 1 µM etoposide (DNA-damaging reagent) for 24 h. MTS tetrazolium compound was added to the cells at 24h post treatment and the number of living cells was measured by reading the absorbance at 490nm. The amount of 490nm absorbance is directly proportional to the number of living cells in culture.

### Statistics

Results of untreated control macrophages and ProTα -treated cells were compared using a one-tailed Student's *t*-test with two samples, assuming equal variance. Experiments were performed in triplicate using cells from a minimum of three different donors, unless otherwise indicated. Data shown are from one representative donor mean ± standard deviation.

### Example 1

### Purification of ProTα variant proteins and their identification

As part of studies to isolate and identify ProTα variant proteins with potent anti-HIV activity, a proteomic study of normal cervicovaginal lavage (CVL) fluids and primary CD8⁺ T cell-secretomes was undertaken. A two-step chromatographic fractionation of the starting material was performed and the fractions were assayed for anti-HIV activity. The proteins in active peaks (with potent anti-HIV activity) were digested with trypsin and tryptic peptides were analyzed by liquid chromatography nanospray/tandem mass spectrometry or LC/MS⁵ (the mass spectrometer is set to analyze the five most abundant peptides in each scan).

Each fraction contained primarily one ProTα variant. However, depending on the different fractionation techniques, the presence of each variant could shift from one fraction to another. Breast Cancer or CD8⁺ ProTα was found in Fractions 2 or 3. CVL-pProTα was found in Fractions 3 or 4. Isoform B ProTα was found in Fractions 4 or 5. Isoform A ProTα was found in Fractions 10.

Unique peptides with excellent spectra (indicating a high degree of reliability in the identifications) identified four ProTα variant proteins, pProTα in CVL, isoA-ProTα and isoB-ProTα in CD8⁺ T cells fractions and ProTα from breast cancer supernatant (shown highlighted in Figure 1A).

The identified peptide sequences are presented below:
From CVL:
   Peptide1: MSDAAVDTSSEITTEDLK (SEQ ID NO: 52)
   Peptide2: DAPANRNANEENGEPEADNEVD (SEQ ID NO: 53)
From CD8⁺ cell conditioned medium:
   Isoform iso-A ProTα
      Peptide1: MPCGARGLSHSR (SEQ ID NO: 54)
      Peptide2: GLSHSRAGAGIAHRV (SEQ ID NO: 55)
   Isoform iso-B ProTα
      Peptide1: EVVEEAENGRDAPANGNAVR (SEQ ID NO: 56)
Breast cancer derived Prothymosin alpha:
   Peptide1: EEEGDGEEEDGDEDEGAES

To amplify mRNAs coding for these proteins, specific primers (Figure 1B, highlighted) were designed based on the coding sequences for the processed pseudogene (pProTα) or the splice variants of cProTα, isoA-ProTα and isoB-ProTα. Reverse transcription polymerase chain reaction (RT-PCR) with primers to pProTα was performed on RNA extracted from CD8, ectocervical and endocervical cells. PCR products were purified using a Qiagen PCR cleanup kit (Qiagen, CA) and analyzed by agarose gel electrophoresis prior to sequencing. Amplified full length pProTα was obtained, and the nucleotide sequence was confirmed by sequence analyses (Figure 1C).

### Example 2

### ProTα variants (pProTα, isoA and isoB) have potent anti-HIV activity

Native proteins present in primary CD8⁺ T cell secretomes or normal cervicovaginal lavage (CVL) fluids were screened for the ability to inhibit HIV-1 replication after viral entry. Primary human monocytes were separated by magnetic beads using a Miltenyi Biotec CD14⁺ cell isolation kit. At day 10 after monocyte differentiation into macrophages, cells were plated at concentrations of 0.2 x 10⁵ to 0.3 x 10⁵ cells per well. Purified primary macrophages were infected by incubating cells with the R5 isolate of HIV-1_{BaL} for 2 hours at a multiplicity of infection (MOI) of either 0.1 or 0.01. The cells were washed with PBS to remove unbound virus. Subsequently, macrophages were treated with one of the following: common cProTα (200ng/ml) in control media, a media plus fraction with a ProTα variant (isoA-ProTα or isoB-ProTα containing fractions derived from CD8⁺ T cell conditioned media or pProTα derived from CVL fluids; total protein concentration was 3ng/ml for each fraction and 95% pure by mass spec analysis), or fraction 1 from CD8⁺ T cell-conditioned medium, which is known to be devoid of ProTα variants. One-half of the medium was replaced every 3 to 4 days with new ProTα and fresh medium. At day 7 post-infection, HIV-1 replication was measured by determining HIV-1 p24 antigen in supernatants of infected cells by enzyme-linked immunosorbent assay (ELISA) (SAIC, Frederick, MD).

To determine the anti-HIV-1 activity of chromatographic fractions containing pProTα, isoA-ProTα and isoB-ProTα (3 ng/ml; >95% pure by mass spec analysis), primary human macrophages were infected for 2 hours with HIV_{BaL} and then washed to remove unbound HIV before treatment with ProTα variants. All three ProTα variants suppressed HIV-1 replication (by more then 95% compared to control) as measured by a decrease in HIV p24 antigen detected by ELISA assay (Figure 2A). An MTS cell viability assay (Promega, CA) demonstrated that treatment with ProTα variants did not induce cellular toxicity (Figure 2B), whereas positive control cells treated with 1µM of etoposide (Sigma) (DNA damaging agent) showed a statistically significant reduction in cell viability (Figure 2B).

Fractions #1,2,3 were derived from cervicovaginal lavage fluids of healthy women and contain pseudogene derived p ProTα (CVL derived pProTα).

### Example 3

### ProTα variants induce IFN-β, type III IFNs (λ1 and λ3) and RANTES expression

Native proteins present in primary CD8⁺ T cell secretomes were tested for the ability to induce IFN and RANTES mRNA in human macrophages after viral entry. Primary human monocytes were separated by magnetic beads using a Miltenyi Biotec CD14⁺ cell isolation kit. At day 10 after monocyte differentiation into macrophages, cells were plated at concentrations of 0.2 x 10⁵ to 0.3 x 10⁵ cells per well. Mouse macrophages were cultured as described in Mosoian, A., Teixeira, A., Burns, C.S., Sander, L.E., Gusella, G.L., He, C., Blander, J.M., Klotman, P., and Klotman, M.E. 2010 107:10178-10183. The cells were treated as specified below.

For detection of type I IFN, primary human macrophages were treated with one of the following for 2 hours: common cProTα (200 ng/ml), media fraction containing isoA-ProTα (3ng/ml, 95% purity), media fraction containing isoB-ProTα (3ng/ml, 95% purity), fraction 1 from CD8⁺ cell-conditioned medium or untreated medium (control).

For detection of type III IFNs (λ1 and λ3), primary human macrophages were treated with the following for 4 hours: cProTα (20 ng/ml), Poly I:C (100µg/ml; positive control) a chemical analog of double stranded RNA, or untreated medium (negative control).

For detection of RANTES, primary human macrophages were treated with the following for 2 hours: cProTα (200ng/ml), fraction 1 from CD8⁺ cell-conditioned medium (3ng/ml), IFN-αl (10 ng/ml; positive control) or untreated medium (control).

For detection of mouse type I IFN-β, wild type mouse macrophages were treated with 3ng/ml of native ProTα variants, isoA or isoB, LPS (10 ng/ml; positive control), or untreated medium (control).

After treatment, RNA was extracted from each sample and the mRNA transcripts of human type I IFN-β, type III IFNs (λ1, λ3) and RANTES was measured by RT-qPCR. Expression of mRNA was normalized to human ribosomal protein 11 (RPS11) control gene and fold induction of mRNA was calculated relative to untreated medium (control). For detection of mouse type I IFN-β mRNA levels, mouse specific IFN-β primers were used in qRT-PCR. Expression of mRNA was normalized to ribosomal protein 11 (Mouse RPS 11) control gene and fold induction of mRNA was calculated relative to untreated medium (control).

The ProTα variants induced expression of type I IFN-β (a labile and transient inducer of IFN-α), type III IFNs λ1 and λ3 (also known as IL-29 and IL-28B, respectively) and RANTES in primary human macrophages as determined by reverse transcription quantitative real time PCR (RT-qPCR) (Figures 3A, 3B and 3C respectively). The combination of IFN-β, type III IFNs λ1 and λ3 and RANTES induced by ProTα variants could lead to a powerful HIV inhibition. RANTES is a ligand for CCR5 and is reported to potently inhibit CCR5-tropic HIV-1 isolates (R5 HIV, e.g. HIV_{BaL}) entry into target cells (22) and its effect is seen best when added prior to infection. In contrast to RANTES, the IFNs are known to suppress HIV at multiple steps post viral entry. Inhibition of HIV replication in human macrophages by ProTα variants, shown in Figure 2A, wherein these experiments were designed to exclude the effect of RANTES on suppression of HIV_{BaL} entry and focus on post-entry events. The ProTα variant isoA induced a more than 300-fold increase in IFN-β mRNA in human macrophages, while isoB generated about a 10-fold less (30-fold) induction. Fraction 1, a separate chromatographic peak fraction from the same fractionation containing an identical amount of protein (3ng/ml), did not induce IFN-β mRNA. To test whether more potent anti-HIV-1 activity of ProTα variants exists in cells exposed to protein before HIV-1 infection, cells were pretreated with ProTα variants and then infected with HIV_{Bal}. In this experiment, primary human macrophages were pretreated (1 hour with fractions at 3 ng/ml) with pProTα, isoA or IsoB and they were then infected with HIVB_{aL}. The concentrations of HIV-1 p24 antigen in the supernatants of infected cells (as a measure of viral replication) at 7 days post-infection were below the level of detection with no cellular toxicity, suggesting a potent synergistic effect of RANTES and IFN-β and type III IFNs (λ1 and λ3) (data not shown). To study the mechanism responsible for type I IFN induction by ProTα variants and to take advantage of the genetically engineered (major TLR and TLR adaptor molecule deficient) mice, ProTα variants were tested for the ability to induce IFN-β activity in wild type mouse macrophages. The data demonstrated that ProTα variant isoB induced IFN-β mRNA (16 fold) in wild type mouse macrophages, while isoA had no IFN-β-inducing activity in these cells (Figure 3D). This suggested that isoA protein is not conserved between human and mouse species. Given that ProTα variants stimulate RANTES, type I and type III IFN production and that variants have a more potent anti-HIV activity when cells are treated before viral infection, suggests a possible synergistic effect of all three anti-HIV proteins could occur simply by applying ProTα variants.

Three previously unreported ProTα variants with potent anti-HIV activity have been isolated and identified. The data also show that new ProTα variants induce innate inhibitors of HIV-1 replication: type I, type III IFNs (λ1, λ3) and RANTES in human macrophages.

### Example 4

### ProTα release is triggered by HIVB_{aL} infection or poly I:C transfection of target cells Induction of ProTα variants

Primary human CD8⁺ T cells were stimulated with Phytohaemaglutinin (5 µg/ml; PHA) or with beads conjugated with anti-CD3 antibody (1:100 dilution from Miltenyi Biotec) to mimic T-cell receptor activation, for 24 hours. As control samples, cells were incubated with media alone or media with IgG (5 µg/ml). RNA was extracted from samples and RT-qPCR was used to detect levels of ProTα variants, common ProTα and IFN-γ mRNA expression.

### Isolation and culture of primary human CD4⁺ cells

Primary CD4⁺ T-cells were isolated from blood as described in Mosoian, A., Teixeira, A., High, A.A., Christian, R.E., Hunt, D.F., Shabanowitz, J., Liu, X., and Klotman, M. 2006. J Virol 80:9200-9206 using Miltenyi biotech isolation kit. Purity of cell population was confirmed to be greater the 95% by FACS analyses.

### Release of ProTα protein from HIV_{BaL} infected CD4⁺ T cells or ectocervical cells

Primary CD4⁺ T-cells were infected with HIV_{BaL} at MOI 0.1 for 2h followed by washes with PBS (to remove unbound virus) and 24 hours post infection supernatant was collected for Elisa assay) or incubated with medium alone. Ectocervical cells were infected with HIV_{BaL} at MOI 0.1, treated with poly I:C (100ug/ml) or incubated with medium alone for 24 hours. Supernatant was collected and used for a human ProTα ELISA assay.

### Human ProTα ELISA

Supernatants from primary human CD4⁺ T cells infected with HIV_{BaL} for 24h were collected, spun down, and used in an ELISA to determine ProTα protein concentrations.

The data demonstrate that stimulation of CD8⁺ T-cells with beads conjugated with anti-CD3 and anti-CD28 antibody (to mimic T-cell receptor activation) selectively upregulated isoB-ProTα mRNA (by 3- fold), while neither cProTα nor CVL-derived pProTα mRNAs were activated. Stimulation of CD8⁺ T cells was detected by IFN gamma induction (used as a positive control), which was upregulated by 15 fold (Figure 4A). Infection of primary CD4⁺ T-cells with HIV_{BaL} (MOI of 0.1) induced a robust, statistically significant release of ProTα (60ng/ml) measured at 24 hours post-infection by an ELISA with an antibody to the N-terminal domain of ProTα (N-terminal domain is similar in a number of ProTα variants (Figure 1A)). These data suggested that one or many of these variants had been induced (Figure 4B). To examine whether activation of cervicovaginal (CV) epithelial cells with HIV_{BaL} could induce release of ProTα, ectocervical cells were infected with HIV_{BaL} for 3 hours followed by PBS washes to remove unbound virus. After 24 hours post-infection, supernatants were collected for ProTα ELISA assays. The data showed that infection of ectocervical cells with HIV_{BaL} or transfection of these cells with the TLR-3 ligand poly I:C (a chemical analog of double stranded RNA) induced a robust statistically significant release of ProTα (450ng/ml and 350ng/ml respectively) using the same ProTα ELISA as described above (Figure 4C). To exclude the possibility that dead cells contributed to the released ProTα detected in the ELISA, MTS cytoxicity assays were performed in parallel on CD4⁺ T-cells or ectocervical cells, in which cells were infected (for 24 hours with HIV_{BaL}; MOI of 0.1) or mock infected. The data showed no difference in cell viability in samples which were mock infected or infected with HIV_{BaL} suggesting that release of ProTα in response to viral infection is not due to cell death (Figure 4D). Conversely, in positive control cells treated with 1µM of etoposide (Sigma) (DNA damaging agent), a statistically significant reduction in MTS cell viability assay was detected (Figure 4D).

ProTα is released in response to HIV-1 infection from a number of cell-types including cervical epithelial cells (ectocervical). In response to TCR activation in CD8⁺ T cells, iso-B ProTα mRNA is upregulated.

### Example 5

### Purification and identification of ProTα variants

The majority of mucosal HIV-1-exposures do not result in productive infections suggesting that the innate immune defenses at mucosal sites are highly protective (23, 24). Studies by a number of groups have shown that high levels of RANTES (CCL5) and interferon-alpha (IFN-α) in cervicovaginal (CV) mucosa correlate with the protection against HIV-1 transmission (25, 26). Other studies have shown that ProTα potently suppresses HIV by stimulating type I IFN production via TLR4 (10, 27). To test whether ProTα protein is present in CV mucosa the presence of ProTα, we tested in three healthy donor cervicovaginal lavages (CVL) fluids by ELISA. The three CVLs all contained considerable levels of ProTα at concentrations of 90 ng/ml, 63 ng/ml and 40 ng/ml.

Next, proteomic studies were conducted to purify and identify proteins with potent anti-HIV activity from CVL fluids of women without sexually transmitted disease and supernatants of primary human CD8⁺ T cell.

Peptides of p7-ProTα and IsoB were identified from CVL and CD8⁺ T cell fractions. Iso B ProTα, an alternatively spliced variant of cProTα gene located on chromosome 2 accession number EAW70973, was identified after sequencing of the human genome (28). p7 ProTα is transcribed from gene located on chromosome 14 and was long considered to be pseudogene accession number AAB08708 (19).

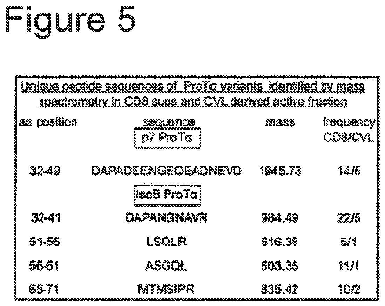

Unique mass spectrometry was identified for each ProTα genetic variant peptide sequences, and the frequencies of presence in the active fractions derived from CD8⁺ T cells and CVL are shown in Figure 5. Unique sequences for p7 genetic variant of ProTα was detected 14 times in active fraction of CD8⁺ T cells and 5 times in CVL. Four different unique peptide sequences corresponding to IsoB variant of ProTα were identified by mass spectrometry from active fractions derived from CD8⁺ T cells and CVL (Figure 5). Mass spectrometry data on active fractions derived from CD8+T cells and CVL suggest presence of novel genetic variants of ProTα p7 and IsoB.

### Example 6

### Native ProTα variants IsoB and p7 suppress HIVB_{aL} replication in primary human macrophages and induce IFN-β, IFN lambda 1 (λ1) and RANTES

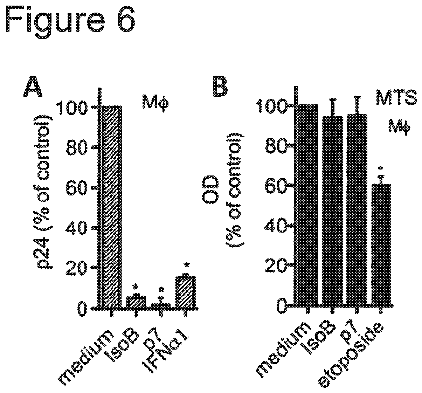

To determine the anti-HIV-1 activity of chromatographic fractions containing p7 ProTα and IsoB ProTα (>95% pure by mass spec analysis) primary human macrophages were infected for 2 h with HIVB_{aL} followed by washes to remove unbound HIV and were further cultured in medium containing 3 ng/ml of each ProTα variant. Both p7 ProTα and IsoB ProTα suppressed HIV-1 replication by more than 95% as measured by HIV p24 ELISA (Figure 6A). This level of suppression was comparable to that exhibited by IFNα1 (Figure 6A). At the concentrations tested, the native ProTα variants exhibited no cellular toxicity, whereas the positive control cells treated with 1 µM of etoposite (DNA damaging agent) a statistically significant reduction in cell viability was observed 3 days post treatment (Figure 6B).

The anti-HIV activity of cProTα has been shown to be type I IFN dependent. To understand whether the genetic variants of ProTα also mediate their anti-HIV-1 activity through type I IFN production, human macrophages were incubated with native IsoB and p7 (3 ng/mL) or LPS as a positive control (100 ng/ml) and measured IFN-β and IFN-λ1 mRNA by real time PCR (RT-qPCR) (Figure 7A). Native IsoB ProTα potently induced (112-fold) IFN-β expression and (60-fold) IFN-λ1 mRNA levels, whereas native p7 ProTα induced IFN-β 276-fold and IFN-λ1 166-fold. An unrelated fraction lacking ProTα but containing the same amount of protein did not upregulate IFN-β and IFN-λ1 expression (Fr1, Figure 7A).

The next question was whether the native ProTα variants (IsoB and p7) also induce pro-inflammatory cytokines, such as, TNF-α and IL-6. Native IsoB ProTα induced 31-fold TNF-α and 54-fold IL-6 mRNA, while p7 ProTα variant induced TNF-α 26-fold and IL-6 102-fold (Figure 7B). We examined the induction of RANTES (a known ligand of the CCR5 receptor and one of the entry receptors used by HIV-1 R5 viruses). Native IsoB ProTα induced 20-fold more RANTES than the mock treated cells, whereas the p7 ProTα variant induced 13-fold (Figure 7C).

### Example 7

### Recombinant ProTα variants IsoB and p7 suppress HIVB_{aL} replication in primary human macrophages and CD4⁺ T cells and induce IFN-β, IFN lambda 1 (λ1) and RANTES

To determine whether anti-HIV-1 and type I and type III-IFN inducing activities of fractions containing genetic variants of ProTα was solely attributed to ProTα and not to other contaminants, cProTα and its variants were cloned and expressed. cProTα, IsoB, p7 and the N-terminal peptide of ProTα -Thymosin alpha 1 (Tα1) genes were cloned in the pRSETA bacterial expression vector (Invitrogen) that produces a fusion with an N-terminal 6 histidine and enterokinase cleavage site. Tα1 the N-terminal of ProTα served as a negative control for possible LPS contamination, because it lacks anti-HIV and IFN-inducing activities (10). After protein purification with Ni-NTA columns and cleavage with enterokinase potential contaminating LPS was also removed by polymyxin columns (10). Western blotting analyses using anti-N-terminal and anti-C-terminal antibodies to cProTα showed that all the ProTα variants were expressed (Figure 8A). Western blotting analyses shows that the recombinant cProTα proteins migrate as monomers and trimers and that Tα1 predominantly migrates as a trimer (∼10kD) and hexamers (∼30kD), which is in agreement with Haritos data (29) (Figure 8A).

Recombinant IsoB ProTα protein mainly migrates as trimers (∼ 33 kD) and has less monomers then cProTα and p7 (Figure 8A). In contrast, recombinant protein p7 ProTα is presented mainly by oligomers (∼10kD) and possibly by trimers (∼33kD) (Figure 8A).

Next the anti-HIV-1 activity of the recombinant ProTα variants was tested by incubating human macrophages and blood derived CD4⁺ T cells with recombinant cProTα, IsoB and p7 (200 ng/mL), the negative control Tα1 (200 ng/ml) and the positive control IFNα1 (100 U/ml).

Recombinant ProTα variants suppressed HIV-1 replication in primary human macrophages by more than 95% as measured by p24 antigen ELISA (Figure 8B). HIV replication in primary CD4⁺ T cells was reduced by the ProTα variants (Figure 8C), which is consistent with our previous work that cProTα had less anti-HIV activity in primary CD4⁺ T cells (38% of control) than in primary macrophages (Figure 8C). Interestingly, isoB and p7 recombinant proteins both have stronger anti-viral activities in primary CD4⁺ T cells and macrophages than cProTα (Figure 8C). Recombinant protein Tα1 which was cloned and purified by the same method did not suppress HIV replication in macrophages (Figure 8B) and CD4⁺ T cells (Figure 8C). Anti-HIV activity of recombinant proteins in macrophages and CD4⁺ T cells was not due to cellular toxicity (Figure 8D). At the concentrations tested, the recombinant ProTα variants exhibited no cellular toxicity, whereas the positive control cells treated with 1 µM of etoposite (DNA damaging agent) resulted in a statistically significant reduction in cell viability (Figure 8D).

As shown above, native ProTα variants IsoB and p7 induced IFN-β, IFN λ1, TNF-α, IL-6 and RANTES expression (Figure 7A, B and C). To test if the different recombinant ProTα proteins induce analogous cytokines and chemokines IFN-β, IFN-λ1, TNF-α, IL-6 and RANTES mRNA levels were measured by RT-qPCR. Similar with the native genetic variants all recombinant ProTα proteins, were strong inducers of IFN-β, IFN-λ1, TNF-α, IL-6 and RANTES expression (Figure 9A, B and C).

Recombinant cProTα induced IFN-β 248-fold and IFN-λ1 50 fold (Fig. 9A). Recombinant IsoB ProTα induced IFN-β mRNA 197-fold and IFN-λ1 mRNA160-fold, while p7 ProTα induced more than three times the amount of IFN-β (637-fold) and almost half the amount of IFN-λ1 (85-fold) mRNAs.

Recombinant cProTα induced TNF-α and IL-6 by 16- and 213-fold, respectively (Figure 9B). Recombinant IsoB ProTα induced TNF-α mRNA 26-fold and IL-6 mRNA 334-fold, while the p7 ProTα variant induced similar levels of TNF-α (24-fold) and IL-6 (306-fold) mRNAs (Figure 9B). Recombinant cProTα induced 4-fold RANTES mRNA (Figure 9C). Recombinant IsoB ProTα induced 14-fold RANTES increase while p7 ProTα variant induced two-fold less (7-fold) (Figure 9C).

Recombinant Tα1 purified by the same method did not induce IFN-β or IFN-λ1 and induced only low levels of TNF-α and IL-6 (7-fold and 10-fold respectively).

Because of the similarities in responses mediated by ProTα and LPS, care was taken to exclude potential contamination of recombinant ProTα with residual levels of LPS. Endotoxin removing gel was used to exclude any LPS in the ProTα protein preparations. The endotoxin levels were measured - which was consistently below the threshold level of <1 EU (endotoxin units) per 1 µg of protein. Together, it is unlikely that the anti-HIV-1 activity (Figure 8B and 8C), induction of IFN-β (Figure 9A), and TNF-α and IL-6 (Figure 9B) were due to contaminating LPS. In addition, recombinant protein Tα1 purified by the same method did not show anti-HIV and type I IFN inducing activities (Figure 8B and Figure 9A, respectively). To further show that the observed effects are caused by ProTα proteins and not by LPS, the ProTα variants (20 ng/ml) were digested with protease and tested their IFN-β mRNA-inducing activities in macrophages. Proteinase digestion of recombinant genetic variants completely abrogated IFN-β induction (Figure 9D), whereas proteinase treatment had no effect on corresponding LPS-mediated activity. These results collectively exclude LPS contamination from our recombinant ProTα preparations and underscore that the responses in macrophages we observed were primarily mediated through genetic variants of ProTα proteins.

Patents, patent applications, publications, product descriptions, and protocols which are cited throughout this application are incorporated herein by reference in their entireties for all purposes.

The embodiments illustrated and discussed in this specification are intended only to teach those skilled in the art the best way known to the inventors to make and use the invention. Nothing in this specification should be considered as limiting the scope of the present invention. Modifications and variation of the above-described embodiments of the invention are possible without departing from the invention, as appreciated by those skilled in the art in light of the above teachings. It is therefore understood that, within the scope of the claims and their equivalents, the invention may be practiced otherwise than as specifically described.

### References

1. Wu, C.G., Habib, N.A., Mitry, R.R., Reitsma, P.H., van Deventer, S.J., and Chamuleau, R.A. 1997. Overexpression of hepatic prothymosin alpha, a novel marker for human hepatocellular carcinoma. Br J Cancer 76:1199-1204.
2. Sasaki, H., Sato, Y., Kondo, S., Fukai, I., Kiriyama, M., Yamakawa, Y., and Fujii, Y. 2001. Expression of the prothymosin alpha mRNA correlated with that of N-myc in neuroblastoma. Cancer Lett 168:191-195.
3. Mori, M., Barnard, G.F., Staniunas, R.J., Jessup, J.M., Steele, G.D., Jr., and Chen, L.B. 1993. Prothymosin-alpha mRNA expression correlates with that of c-myc in human colon cancer. Oncogene 8:2821-2826.
4. Baxevanis, C.N., Sfagos, C., Anastasopoulos, E., Reclos, G.J., and Papamichail, M. 1990. Prothymosin-alpha enhances HLA-DR antigen expression on monocytes from patients with multiple sclerosis. J Neuroimmunol 27:141-147.
5. Baxevanis, C.N., Thanos, D., Reclos, G.J., Anastasopoulos, E., Tsokos, G.C., Papamatheakis, J., and Papamichail, M. 1992. Prothymosin alpha enhances human and murine MHC class II surface antigen expression and messenger RNA accumulation. J Immunol 148:1979-1984.
6. Skopeliti, M., Kratzer, U., Altenberend, F., Panayotou, G., Kalbacher, H., Stevanovic, S., Voelter, W., and Tsitsilonis, O.E. 2007. Proteomic exploitation on prothymosin alpha-induced mononuclear cell activation. Proteomics 7:1814-1824.
7. Bowick, G.C., Soman, K.V., Wang, H., Aronson, J.F., Luxon, B.A., Lomas, L.O., Gorenstein, D.G., and Herzog, N.K. 2010. Proteomic analysis of Pichinde virus infection identifies differential expression of prothymosin-alpha. JBiomed Biotechnol 2010.
8. Mosoian, A., Teixeira, A., High, A.A., Christian, R.E., Hunt, D.F., Shabanowitz, J., Liu, X., and Klotman, M. 2006. Novel function of prothymosin alpha as a potent inhibitor of human immunodeficiency virus type 1 gene expression in primary macrophages. J Virol 80:9200-9206.
9. Romani, L., Bistoni, F., Gaziano, R., Bozza, S., Montagnoli, C., Perruccio, K., Pitzurra, L., Bellocchio, S., Velardi, A., Rasi, G., et al. 2004. Thymosin alpha 1 activates dendritic cells for antifungal Th1 resistance through toll-like receptor signaling. Blood 103:4232-4239.
10. Mosoian, A., Teixeira, A., Burns, C.S., Sander, L.E., Gusella, G.L., He, C., Blander, J.M., Klotman, P., and Klotman, M.E. 2010. Prothymosin-alpha inhibits HIV-1 via Toll-like receptor 4-mediated type I interferon induction. Proc Natl Acad Sci U S A 107:10178-10183.
11. Mosoian, A. 2011. Intracellular and extracellular cytokine-like functions of prothymosin alpha: implications for the development of immunotherapies. Future Med Chem 3:1199-1208.
12. Grunberg, E., Eckert, K., Maurer, H.R., Immenschuh, P., and Kreuser, E.D. 1997. Prothymosin alpha1 effects on IL-2-induced expression of LFA-1 on lymphocytes and their adhesion to human umbilical vein endothelial cells. J Interferon Cytokine Res 17:159-165.
13. Skopeliti, M., Iconomidou, V.A., Derhovanessian, E., Pawelec, G., Voelter, W., Kalbacher, H., Hamodrakas, S.J., and Tsitsilonis, O.E. 2008. Prothymosin alpha immunoactive carboxyl-terminal peptide TKKQKTDEDD stimulates lymphocyte reactions, induces dendritic cell maturation and adopts a beta-sheet conformation in a sequence-specific manner. Mol Immunol.
14. Voutsas, I.F., Baxevanis, C.N., Gritzapis, A.D., Missitzis, I., Stathopoulos, G.P., Archodakis, G., Banis, C., Voelter, W., and Papamichail, M. 2000. Synergy between interleukin-2 and prothymosin alpha for the increased generation of cytotoxic T lymphocytes against autologous human carcinomas. Cancer Immunol Immunother 49:449-458.
15. Matsunaga, H., and Ueda, H. 2010. Stress-induced non-vesicular release of prothymosin-alpha initiated by an interaction with S100A13, and its blockade by caspase-3 cleavage. Cell Death Differ*.*
16. Evstafieva, A.G., Belov, G.A., Kalkum, M., Chichkova, N.V., Bogdanov, A.A., Agol, V.I., and Vartapetian, A.B. 2000. Prothymosin alpha fragmentation in apoptosis. FEBS Lett 467:150-154.
17. Adams, M.D., Kerlavage, A.R., Fleischmann, R.D., Fuldner, R.A., Bult, C.J., Lee, N.H., Kirkness, E.F., Weinstock, K.G., Gocayne, J.D., White, O., et al. 1995. Initial assessment of human gene diversity and expression patterns based upon 83 million nucleotides of cDNA sequence. Nature 377:3-174.
18. Pineiro, A., Cordero, O.J., and Nogueira, M. 2000. Fifteen years of prothymosin alpha: contradictory past and new horizons. Peptides 21:1433-1446.
19. Rubtsov Iu, P., and Vartapetian, A.B. 1995. [New intronless members of human prothymosin alpha genes]. Mol Biol (Mosk) 29:1320-1325.
20. Manrow, R.E., Leone, A., Krug, M.S., Eschenfeldt, W.H., and Berger, S.L. 1992. The human prothymosin alpha gene family contains several processed pseudogenes lacking deleterious lesions. Genomics 13:319-331.
21. Fichorova, R.N., Rheinwald, J.G., and Anderson, D.J. 1997. Generation of papillomavirus-immortalized cell lines from normal human ectocervical, endocervical, and vaginal epithelium that maintain expression of tissue-specific differentiation proteins. Biol Reprod 57:847-855.
22. Cocchi, F., DeVico, A.L., Garzino-Demo, A., Arya, S.K., Gallo, R.C., and Lusso, P. 1995. Identification of RANTES, MIP-1 alpha, and MIP-1 beta as the major HIV-suppressive factors produced by CD8+ T cells. Science 270:1811-1815.
23. Gray, R. H., M. J. Wawer, R. Brookmeyer, N. K. Sewankambo, D. Serwadda, F. Wabwire-Mangen, T. Lutalo, X. Li, T. vanCott, and T. C. Quinn. 2001. Probability of HIV-1 transmission per coital act in monogamous, heterosexual, HIV-1-discordant couples in Rakai, Uganda. Lancet 357:1149-53.
24. Pope, M., and A. T. Haase. 2003. Transmission, acute HIV-1 infection and the quest for strategies to prevent infection. Nat Med 9:847-52.
25. Hirbod, T., J. Nilsson, S. Andersson, C. Uberti-Foppa, D. Ferrari, M. Manghi, J. Andersson, L. Lopalco, and K. Broliden. 2006. Upregulation of interferon-alpha and RANTES in the cervix of HIV-1-seronegative women with high-risk behavior. J Acquir Immune Defic Syndr 43:137-43.
26. Iqbal, S. M., T. B. Ball, J. Kimani, P. Kiama, P. Thottingal, J. E. Embree, K. R. Fowke, and F. A. Plummer. 2005. Elevated T cell counts and RANTES expression in the genital mucosa of HIV-1-resistant Kenyan commercial sex workers. J Infect Dis 192:728-38.
27. Mosoian, A., A. Teixeira, C. S. Burns, G. Khitrov, W. Zhang, L. Gusella, P. Klotman, and M. Klotman. 2007. Influence of prothymosin-alpha on HIV-1 target cells. Ann N Y Acad Sci 1112:269-85.
28. Venter, J. C., M. D. Adams, E. W. Myers, P. W. Li, R. J. Mural, G. G. Sutton, H. O. Smith, M. Yandell, C. A. Evans, R. A. Holt, J. D. Gocayne, P. Amanatides, R. M. Ballew, D. H. Huson, J. R. Wortman, Q. Zhang, C. D. Kodira, X. H. Zheng, L. Chen, M. Skupski, G. Subramanian, P. D. Thomas, J. Zhang, G. L. Gabor Miklos, C. Nelson, S. Broder, A. G. Clark, J. Nadeau, V. A. McKusick, N. Zinder, A. J. Levine, R. J. Roberts, M. Simon, C. Slayman, M. Hunkapiller, R. Bolanos, A. Delcher, I. Dew, D. Fasulo, M. Flanigan, L. Florea, A. Halpern, S. Hannenhalli, S. Kravitz, S. Levy, C. Mobarry, K. Reinert, K. Remington, J. Abu-Threideh, E. Beasley, K. Biddick, V. Bonazzi, R. Brandon, M. Cargill, I. Chandramouliswaran, R. Charlab, K. Chaturvedi, Z. Deng, V. Di Francesco, P. Dunn, K. Eilbeck, C. Evangelista, A. E. Gabrielian, W. Gan, W. Ge, F. Gong, Z. Gu, P. Guan, T. J. Heiman, M. E. Higgins, R. R. Ji, Z. Ke, K. A. Ketchum, Z. Lai, Y. Lei, Z. Li, J. Li, Y. Liang, X. Lin, F. Lu, G. V. Merkulov, N. Milshina, H. M. Moore, A. K. Naik, V. A. Narayan, B. Neelam, D. Nusskern, D. B. Rusch, S. Salzberg, W. Shao, B. Shue, J. Sun, Z. Wang, A. Wang, X. Wang, J. Wang, M. Wei, R. Wides, C. Xiao, C. Yan, et al. 2001. The sequence of the human genome. Science 291:1304-51.
29. Haritos, A. A., G. J. Goodall, and B. L. Horecker. 1984. Prothymosin alpha: isolation and properties of the major immunoreactive form of thymosin alpha 1 in rat thymus. Proc Natl Acad Sci U S A 81:1008-11.

## Claims

1. An isolated prothymosin alpha polypeptide, wherein the polypeptide comprises:
a) an amino acid sequence that is at least about 95% identical to an amino acid sequence selected from the group consisting of SEQ ID NO: 4, SEQ ID NO: 3, SEQ ID NO: 1, SEQ ID NO: 2, SEQ ID NO: 9, SEQ ID NO:10, SEQ ID NO: 11, SEQ ID NO: 12, and SEQ ID NO: 13;
b) a functional derivative thereof; or
c) a fragment thereof.

2. The isolated prothymosin alpha polypeptide according to claim 1, wherein the polypeptide comprises an amino acid sequence selected from the group consisting of SEQ ID NO: 4, SEQ ID NO: 3, SEQ ID NO: 1, SEQ ID NO: 2, SEQ ID NO: 9, SEQ ID NO:10, SEQ ID NO: 11, SEQ ID NO: 12, and SEQ ID NO: 13.

3. An isolated nucleic acid that encodes a prothymosin alpha polypeptide, wherein the nucleic acid has a nucleotide sequence that is at least about 95% identical to a nucleotide sequence selected from the group consisting of SEQ ID NO: 8, SEQ ID NO: 7, SEQ ID NO: 5, SEQ ID NO: 6, SEQ ID NO: 14, SEQ ID NO: 15, SEQ ID NO: 16, SEQ ID NO: 17, and SEQ ID NO: 18.

4. The isolated nucleic acid according to claim 3, wherein the nucleic acid comprises a nucleotide sequence selected from the group consisting of SEQ ID NO: 8, SEQ ID NO: 7, SEQ ID NO: 5, SEQ ID NO: 6, SEQ ID NO: 14, SEQ ID NO: 15, SEQ ID NO: 16, SEQ ID NO: 17, and SEQ ID NO: 18.

5. A polypeptide according to claim 1 or claim 2 for use in a method for treating or preventing a viral infection, comprising administering the polypeptide to a subject in need thereof in an amount effective to treat or prevent viral infection.

6. The polypeptide for use according to claim 5, wherein the viral infection is caused by a virus selected from the group consisting of Human Immunodeficiency Virus-1 (HIV-1), Human Immunodeficiency Virus-2 (HIV-2), Hepatatis C Virus, Hepatatis B Virus, Hepatatis A Virus, Ebola, Marburg, Dengue viruses, Hemorrhagic Fever Viruses (VHFs), Lassa virus (LASV), Crimean-Congo hemorrhagic fever virus (CCHFV), Rift Valley fever virus (RVFV), and yellow fever virus (YFV), and wherein the viral infection is optionally selected from the group consisting of HIV-1 and HIV-2 infection.

7. A polypeptide according to claim 1 or claim 2 for use in a method for potentiating an immune response, comprising administering the polypeptide to a subject in need thereof, whereby the immune response is potentiated.

8. The polypeptide for use according to claim 7, wherein:
(a) the subject has a viral infection and the immune response to the virus is potentiated;
(b) the subject has a fungal infection and the immune response to the fungus is potentiated;
(c) the subject has a bacterial infection and the immune response to the bacteria is potentiated; or
(d) the subject has a parasitic infection and the immune response to the parasite is potentiated.

9. The polypeptide for use according to claim 7, wherein the method for potentiating an immune response comprises administering the polypeptide to a subject that is undergoing cancer treatment, whereby the immune response to the cancer is potentiated, and optionally wherein the cancer treatment is a treatment with a chemotherapeutic agent.

10. A polypeptide according to claim 1 or claim 2 for use in a method of treating or preventing ischemia, comprising administering to a subject in need thereof in an amount effective to treat or prevent ischemia.

11. A polypeptide according to claim 1 or claim 2 for use in a method for inducing interferons, comprising administering the polypeptide to a subject in need of interferon induction, whereby interferons are induced.

12. The method of claim 11, wherein:
(a) the interferons are selected from the group consisting of type I and type III interferons; or
(b) the subject in need of type I interferon induction has a blood disorder, cancer, a viral infection, or a combination thereof, wherein the blood disorder is optionally leukemia.

13. A kit for potentiating an immune response, comprising:
the polypeptides of claims 1 or 2, and
instructions for use.

14. A pharmaceutical composition comprising:
(a) the polypeptide of claims 1 or 2 and a pharmaceutically acceptable carrier;
(b) the polypeptide of claims 1 or 2 and a chemotherapeutic agent, wherein optionally the composition:
(i) further comprises interferon alpha; or
(ii) the chemotherapeutic agent is selected from the group consisting of dacarbazine, tamoxifen, raloxifene, megestrol, flutamide cyclophosphamide, temozolomide, cisplatin, and paclitaxel;
(c) the polypeptide of claims 1 or 2 and a pegylated interferon-alpha2a; or
(d) an antigen, the polypeptide of claims 1 or 2 and a pharmaceutically acceptable carrier.

15. A polypeptide according to claim 1 or claim 2 for use in a method for inducing RANTES, comprising administering the polypeptide to a subject in need of RANTES induction, whereby RANTES is induced.


SEQUENCE LISTING

<110> ICAHN SCHOOL OF MEDICINE AT MOUNT SINAI

<120> VARIANTS OF PROTHYMOSIN ALPHA AND METHODS OF USING SAME

<130> 242096.000018

<140> PCT/US2013/028717
<141> 2013-03-01

<150> 61/606,193
<151> 2012-03-02

<160> 71

<170> PatentIn version 3.5

<210> 1
<211> 108
<212> PRT
<213> Homo sapiens

<400> 1

<210> 2
<211> 101
<212> PRT
<213> Homo sapiens

<400> 2
Met Pro Cys Gly Ala Arg Gly Leu Ser His Ser Arg Ala Gly Ala Gly
1               5                   10                  15

Ile Ala His Arg Val Arg Leu Ile His Glu Ser Pro Gln Gln Asn Ser

20                  25                  30

Cys Gly Ser Ala Arg Pro Glu Arg Ala Arg Ala Pro Leu Pro Arg Ala
        35                  40                  45

Leu Gly Pro Ala Glu Thr Arg Gln Thr Pro Ala Leu Asn Asp Ser Ala
    50                  55                  60

Ile Gly Asp Cys Ala Ala Arg Asp Ala Pro Gly His Cys Arg Phe Lys
65                  70                  75                  80

His Ala Cys Pro Leu Phe Gly Ile Glu Pro Met Ser Glu Arg Pro Leu
                85                  90                  95

Pro Ala Leu Ser Gln
            100

<210> 3
<211> 86
<212> PRT
<213> Homo sapiens

<400> 3
Met Ser Asp Ala Ala Val Asp Thr Ser Ser Glu Ile Thr Thr Lys Asp
1               5                   10                  15

Leu Lys Glu Lys Lys Glu Val Val Glu Glu Ala Glu Asn Gly Arg Asp
            20                  25                  30

Ala Pro Ala Asn Gly Asn Ala Val Arg Lys Arg Met Glu Met Lys Met
        35                  40                  45

Arg Lys Leu Ser Gln Leu Arg Ala Ser Gly Gln Leu Lys Met Met Arg
    50                  55                  60

Met Thr Met Ser Ile Pro Arg Ser Arg Arg Pro Thr Arg Met Thr Arg
65                  70                  75                  80

Gln Gln Lys Arg Lys Ser
                85

<210> 4
<211> 73
<212> PRT
<213> Homo sapiens

<400> 4

<210> 5
<211> 331
<212> DNA
<213> Homo sapiens

<400> 5
atgtcagacg cagccgtaga caccagctcc gaaatcacca ccgaggactt aaaggagaag      60

aaggaagttg tggaagaggc ggaaaatgga agagacgccc ctgctaacag gaatgctaat     120

gaggaaaatg gggagccgga ggctgacaac gaggtagatg aagaagagga agaaggtggg     180

gaggaagagg aggaggaaga aggtgatggt gaggaagagg atggagatga agatgaggga     240

gctgagtcag ctacgggcaa gcgggcagct gaagatgatg aggatgacga tgtcgatacc     300

cagaagtcag aagaccgacg aggatgacta g                                    331

<210> 6
<211> 303
<212> DNA
<213> Homo sapiens

<400> 6
atgccgtgcg gggcccgcgg gctgtcccac tcgagggctg gagccggcat cgcgcatcga      60

gtccgcttga tacatgaatc cccccagcaa aacagctgcg ggagcgcgcg tccggaacgc     120

gctcgcgcac ccctcccccg cgccctcggc cctgctgaga cccggcaaac tccggccttg     180

aatgacagtg cgatcggcga ctgcgcagcg cgggacgcgc cggggcactg ccgctttaag     240

cacgcttgtc cattgttcgg aatcgagcca atgagcgagc gcccgctccc tgcgctcagc     300

caa                                                                   303

<210> 7
<211> 262
<212> DNA
<213> Homo sapiens

<400> 7
atgtcagacg cagccgtaga caccagctcc gaaatcacca ccaaggactt aaaggagaag      60

aaggaagttg tggaagaggc agaaaatgga agagacgccc ctgctaacgg gaatgctgtg     120

aggaagagga tggagatgaa gatgaggaag ctgagtcagc tacgggcaag cgggcagctg 180

aagatgatga ggatgacgat gtcgatacca agaagcagaa gaccgacgag gatgactaga 240

cagcaaaaaa ggaaaagtta aa 262

<210> 8
<211> 322
<212> DNA
<213> Homo sapiens

<400> 8
atgtcagacg cagccgtaga caccagctcc gaaatcacca ccaaggactt aaaggagaag 60

aaggaagttg tggaagaggc ggaaaatgga agagacgccc ctgctgacga ggaaaatggg 120

gagcaagagg ctgacaatga agtagatgaa gaacaggaag aaggtgggga ggaagaggag 180

gaggaagaag aaggtgaggg tgaggaagaa ggatggagat gaagatgagg aagctgagtc 240

agctaccggc aagtgggcag ctgaagatga tgaggatgac gatgttgata ccaagaagca 300

gaagaccgac gaggatgact ag 322

<210> 9
<211> 114
<212> PRT
<213> Homo sapiens

<400> 9

<210> 10
<211> 117
<212> PRT
<213> Homo sapiens

<400> 10
Met Ser Asp Ala Ala Val Asp Thr Ser Ser Glu Ile Thr Thr Glu Asp
1               5                   10                  15

Leu Lys Glu Lys Lys Glu Val Val Glu Glu Ala Glu Asn Gly Arg Asp
            20                  25                  30

Ala Pro Ala His Gly Asn Ala Asn Glu Glu Asn Gly Glu Pro Asp Asp
        35                  40                  45

Asn Glu Val Asp Glu Glu Glu Glu Glu Gly Gly Glu Glu Glu Glu Glu
    50                  55                  60

Glu Glu Gly Asp Gly Glu Glu Glu Asp Gly Asp Glu Asp Glu Gly Ala
65                  70                  75                  80

Glu Ser Ala Thr Gly Lys Arg Ala Ala Glu Asp Asp Glu Asp Asn Asp
                85                  90                  95

Val Asp Thr Gln Lys Gln Lys Thr Asp Glu Asp Asp Gln Thr Ala Lys
            100                 105                 110

Lys Glu Lys Leu Asn
        115

<210> 11
<211> 64
<212> PRT
<213> Homo sapiens

<400> 11
Met Ser Asp Ala Ala Val Asp Thr Ser Ser Glu Ile Thr Thr Lys Glu
1               5                   10                  15

Glu Glu Glu Glu Glu Glu Gly Asp Gly Glu Glu Glu Asp Gly Asp Glu
            20                  25                  30

Asp Glu Glu Ala Glu Ser Ala Thr Gly Lys Arg Ala Ala Glu Asp Asp
        35                  40                  45

Glu Asp Asp Asp Val Asp Thr Lys Lys Gln Lys Thr Asp Glu Asp Asp
    50                  55                  60

<210> 12

<211> 114
<212> PRT
<213> Homo sapiens

<400> 12
Met Ser Asp Ala Ala Val Asp Thr Ser Ser Glu Ile Thr Thr Lys Asp
1               5                   10                  15

Leu Lys Glu Lys Lys Glu Val Val Glu Glu Ala Glu Asn Gly Arg Asp
            20                  25                  30

Ala Pro Ala Asn Gly Asn Ala Asn Glu Glu Asn Gly Glu Gln Glu Ala
        35                  40                  45

Asp Asn Glu Val Asp Glu Glu Glu Glu Glu Gly Gly Glu Glu Glu Glu
    50                  55                  60

Glu Glu Glu Glu Gly Asp Gly Glu Glu Glu Asp Gly Asp Glu Asp Glu
65                  70                  75                  80

Glu Ala Glu Ser Ala Thr Gly Lys Arg Ala Ala Glu Asp Asp Glu Val
                85                  90                  95

Gly Ser Gly Leu Arg Arg Arg Gly Val Trp His Leu Gly Leu Pro Thr
            100                 105                 110

Cys Leu



<210> 13
<211> 99
<212> PRT
<213> Homo sapiens

<400> 13
Met Ser Asp Ala Ala Val Asp Thr Ser Ser Glu Ile Thr Thr Lys Asp
1               5                   10                  15

Leu Lys Glu Lys Lys Glu Val Val Glu Glu Ala Glu Asn Gly Arg Asp
            20                  25                  30

Ala Pro Ala Asn Gly Asn Ala Asn Glu Glu Asn Gly Glu Gln Glu Ala
        35                  40                  45

Asp Asn Glu Val Asp Glu Glu Glu Glu Glu Gly Gly Glu Glu Glu Glu
    50                  55                  60

Glu Glu Glu Glu Gly Asp Gly Glu Glu Glu Asp Gly Asp Glu Asp Glu
65                  70                  75                  80

Glu Ala Glu Ser Ala Thr Gly Lys Arg Ala Ala Glu Asp Asp Glu Ser
                85                  90                  95

Phe Ser Ala

<210> 14
<211> 345
<212> DNA
<213> Homo sapiens

<400> 14
atgtcagacg cagccgtaga caccagctcc gaaatcacca ccgaggactt aaaggagaag      60

aaggaagttg tggaagaggc ggaaaatgga agagacgccc ctgctcacgg gaatgctaat     120

gaggaaaatg gggagccgga ggctgacaac gaggtagatg aagaagagga agaaggtggg     180

gaggaagaag gtgatggtga ggaagaggat ggagatgaag atgagggagc tgagtcagct     240

acgggcaagc gggcagctga agatgatgag gataacgatg tcgataccca gaagcagaag     300

accgacgagg atgaccagac ggcaaaaaag gaaaagttaa actaa                     345

<210> 15
<211> 354
<212> DNA
<213> Homo sapiens

<400> 15
atgtcagacg cagccgtaga caccagctcc gaaatcacca ccgaggactt aaaggagaag      60

aaggaagttg tggaagaggc ggaaaatgga agagacgccc ctgctcacgg gaatgctaat     120

gaggaaaatg gggagccgga tgacaacgag gtagatgaag aagaggaaga aggtggggag     180

gaagaggagg aggaagaagg tgatggtgag gaagaggacg gagatgaaga tgagggagct     240

gagtcagcta cgggcaagcg ggcagctgaa gatgatgagg ataacgatgt cgatacccag     300

aagcagaaga ccgacgagga tgaccagaca gcaaaaaagg aaaagttaaa ctaa           354

<210> 16
<211> 195
<212> DNA
<213> Homo sapiens

<400> 16
atgtcagacg cagccgtaga caccagctcc gaaatcacca ccaaggaaga ggaggaggaa      60

gaagaaggtg atggtgagga agaggatgga gatgaagatg aggaagctga gtcagctacg     120

ggcaagcggg cagctgaaga tgatgaggat gacgatgtcg ataccaagaa gcagaagacc     180

gacgaggatg actag                                                      195

<210> 17
<211> 345

<212> DNA
<213> Homo sapiens

<400> 17
atgtcagacg cagccgtaga caccagctcc gaaatcacca ccaaggactt aaaggagaag 60

aaggaagttg tggaagaggc agaaaatgga agagacgccc ctgctaacgg gaatgctaat 120

gaggaaaatg gggagcagga ggctgacaat gaggtagacg aagaagagga agaaggtggg 180

gaggaagagg aggaggaaga agaaggtgat ggtgaggaag aggatggaga tgaagatgag 240

gaagctgagt cagctacggg caagcgggca gctgaagatg atgaggtggg ttctggcttg 300

agaagaaggg gggtttggca tctgggtctc cccacctgcc tttag 345


<210> 18
<211> 300
<212> DNA
<213> Homo sapiens

<400> 18
atgtcagacg cagccgtaga caccagctcc gaaatcacca ccaaggactt aaaggagaag 60

aaggaagttg tggaagaggc agaaaatgga agagacgccc ctgctaacgg gaatgctaat 120

gaggaaaatg gggagcagga ggctgacaat gaggtagacg aagaagagga agaaggtggg 180

gaggaagagg aggaggaaga agaaggtgat ggtgaggaag aggatggaga tgaagatgag 240

gaagctgagt cagctacggg caagcgggca gctgaagatg atgagtcttt ctctgcttag 300


<210> 19
<211> 21
<212> DNA
<213> Artificial Sequence

<220>
<223> Description of Artificial Sequence: Synthetic
      primer

<400> 19
atgtcagacg cagccgtaga c 21


<210> 20
<211> 21
<212> DNA
<213> Artificial Sequence

<220>
<223> Description of Artificial Sequence: Synthetic
      primer

<400> 20
cgttagcagg ggcgtctctt c 21


<210> 21
<211> 24
<212> DNA

<213> Artificial Sequence

<220>
<223> Description of Artificial Sequence: Synthetic
      primer

<400> 21
cttcctcctc ctcttcctcc ccac                                              24


<210> 22
<211> 24
<212> DNA
<213> Artificial Sequence

<220>
<223> Description of Artificial Sequence: Synthetic
      primer

<400> 22
gtggggagga agaggaggag gaag                                              24


<210> 23
<211> 26
<212> DNA
<213> Artificial Sequence

<220>
<223> Description of Artificial Sequence: Synthetic
      primer

<400> 23
ctagtcatcc tcgtcggtct tctgac                                            26


<210> 24
<211> 24
<212> DNA
<213> Artificial Sequence

<220>
<223> Description of Artificial Sequence: Synthetic
      primer

<400> 24
aatgttcatg tgcccatcgc agtc                                              24


<210> 25
<211> 24
<212> DNA
<213> Artificial Sequence

<220>
<223> Description of Artificial Sequence: Synthetic
      primer

<400> 25
gaggcggatg aggaacaatg caaa                                              24


<210> 26

<211> 21
<212> DNA
<213> Artificial Sequence

<220>
<223> Description of Artificial Sequence: Synthetic
      primer

<400> 26
atgtcagacg cagccgtaga c                                                21


<210> 27
<211> 26
<212> DNA
<213> Artificial Sequence

<220>
<223> Description of Artificial Sequence: Synthetic
      primer

<400> 27
gctaacggga atgctgtgag gaagag                                           26


<210> 28
<211> 26
<212> DNA
<213> Artificial Sequence

<220>
<223> Description of Artificial Sequence: Synthetic
      primer

<400> 28
ctcttcctca cagcattccc gttagc                                           26


<210> 29
<211> 24
<212> DNA
<213> Artificial Sequence

<220>
<223> Description of Artificial Sequence: Synthetic
      primer

<400> 29
   tttaactttt ccttttttgc tgtc                                              24


<210> 30
<211> 22
<212> DNA
<213> Artificial Sequence

<220>
<223> Description of Artificial Sequence: Synthetic
      primer

<400> 30
cttcctcccc accttcttcc tc                                               22

<210> 31
<211> 22
<212> DNA
<213> Artificial Sequence

<220>
<223> Description of Artificial Sequence: Synthetic
      primer

<400> 31
gaggaagaag gtggggagga ag                                                22


<210> 32
<211> 19
<212> DNA
<213> Artificial Sequence

<220>
<223> Description of Artificial Sequence: Synthetic
      primer

<400> 32
tcatccgcct ccttgctcg                                                    19


<210> 33
<211> 24
<212> DNA
<213> Artificial Sequence

<220>
<223> Description of Artificial Sequence: Synthetic
      primer

<400> 33
attctcatgg tttgggttgg gtgg                                              24


<210> 34
<211> 20
<212> DNA
<213> Artificial Sequence

<220>
<223> Description of Artificial Sequence: Synthetic
      primer

<400> 34
gccgagacta tctgcactac                                                   20


<210> 35
<211> 20
<212> DNA
<213> Artificial Sequence

<220>
<223> Description of Artificial Sequence: Synthetic
      primer

<400> 35

atgtccagcc tcagaacttc                                                    20

<210> 36
<211> 20
<212> DNA
<213> Artificial Sequence

<220>
<223> Description of Artificial Sequence: Synthetic
      primer

<400> 36
gtcagagtgg aaatcctaag                                                    20

<210> 37
<211> 20
<212> DNA
<213> Artificial Sequence

<220>
<223> Description of Artificial Sequence: Synthetic
      primer

<400> 37
acagcatctg ctggttgaag                                                    20

<210> 38
<211> 24
<212> DNA
<213> Artificial Sequence

<220>
<223> Description of Artificial Sequence: Synthetic
      primer

<400> 38
acagcctcag agtgtttctt ctgc                                               24

<210> 39
<211> 24
<212> DNA
<213> Artificial Sequence

<220>
<223> Description of Artificial Sequence: Synthetic
      primer

<400> 39
acagggactt gaactgggct atgt                                               24

<210> 40
<211> 24
<212> DNA
<213> Artificial Sequence

<220>
<223> Description of Artificial Sequence: Synthetic
      primer

<400> 40
acctcaaata tgtggccgat ggga                                              24


<210> 41
<211> 24
<212> DNA
<213> Artificial Sequence

<220>
<223> Description of Artificial Sequence: Synthetic
      primer

<400> 41
aaggagtagg gctcagcgca taaa                                              24


<210> 42
<211> 20
<212> DNA
<213> Artificial Sequence

<220>
<223> Description of Artificial Sequence: Synthetic
      primer

<400> 42
ctgaatgact tggaagcctg                                                   20


<210> 43
<211> 20
<212> DNA
<213> Artificial Sequence

<220>
<223> Description of Artificial Sequence: Synthetic
      primer

<400> 43
atttctgctc tgacaacctc                                                   20


<210> 44
<211> 19
<212> DNA
<213> Artificial Sequence

<220>
<223> Description of Artificial Sequence: Synthetic
      primer

<400> 44
tccaacccag cagtcgtct                                                    19


<210> 45
<211> 19
<212> DNA
<213> Artificial Sequence

<220>

<223> Description of Artificial Sequence: Synthetic
      primer

<400> 45
ttggcggttc tttcgggtg                                                    19


<210> 46
<211> 20
<212> DNA
<213> Artificial Sequence

<220>
<223> Description of Artificial Sequence: Synthetic
      primer

<400> 46
agatgtcctc aactgctctc                                                   20


<210> 47
<211> 20
<212> DNA
<213> Artificial Sequence

<220>
<223> Description of Artificial Sequence: Synthetic
      primer

<400> 47
agattcacta ccagtcccag                                                   20


<210> 48
<211> 20
<212> DNA
<213> Artificial Sequence

<220>
<223> Description of Artificial Sequence: Synthetic
      primer

<400> 48
cgtgacgaag atgaagatgc                                                   20


<210> 49
<211> 20
<212> DNA
<213> Artificial Sequence

<220>
<223> Description of Artificial Sequence: Synthetic
      primer

<400> 49
gcacattgaa tcgcacagtc                                                   20


<210> 50
<211> 20
<212> DNA
<213> Artificial Sequence

<220>
<223> Description of Artificial Sequence: Synthetic
      primer

<400> 50
aggcaaggct atgtgattac                                                    20


<210> 51
<211> 20
<212> DNA
<213> Artificial Sequence

<220>
<223> Description of Artificial Sequence: Synthetic
      primer

<400> 51
gctatgtttt catcagggtc                                                    20


<210> 52
<211> 18
<212> PRT
<213> Homo sapiens

<400> 52
Met Ser Asp Ala Ala Val Asp Thr Ser Ser Glu Ile Thr Thr Glu Asp
1               5                   10                  15


Leu Lys




<210> 53
<211> 22
<212> PRT
<213> Homo sapiens

<400> 53
Asp Ala Pro Ala Asn Arg Asn Ala Asn Glu Glu Asn Gly Glu Pro Glu
1               5                   10                  15


Ala Asp Asn Glu Val Asp
            20


<210> 54
<211> 12
<212> PRT
<213> Homo sapiens

<400> 54
Met Pro Cys Gly Ala Arg Gly Leu Ser His Ser Arg
1               5                   10


<210> 55
<211> 15
<212> PRT

<213> Homo sapiens

<400> 55
Gly Leu Ser His Ser Arg Ala Gly Ala Gly Ile Ala His Arg Val
1               5                   10                  15


<210> 56
<211> 20
<212> PRT
<213> Homo sapiens

<400> 56
Glu Val Val Glu Glu Ala Glu Asn Gly Arg Asp Ala Pro Ala Asn Gly
1               5                   10                  15


Asn Ala Val Arg
            20


<210> 57
<211> 110
<212> PRT
<213> Homo sapiens

<400> 57
Met Ser Asp Ala Ala Val Asp Thr Ser Ser Glu Ile Thr Thr Lys Asp
1               5                   10                  15


Leu Lys Glu Lys Lys Glu Val Val Glu Glu Ala Glu Asn Gly Arg Asp
            20                  25                  30


Ala Pro Ala Asn Gly Asn Ala Asn Glu Glu Asn Gly Glu Gln Glu Ala
        35                  40                  45


Asp Asn Glu Val Asp Glu Glu Glu Glu Glu Gly Gly Glu Glu Glu Glu
    50                  55                  60


Glu Glu Glu Glu Gly Asp Gly Glu Glu Glu Asp Gly Asp Glu Asp Glu
65                  70                  75                  80


Glu Ala Glu Ser Ala Thr Gly Lys Arg Ala Ala Glu Asp Asp Glu Asp
                85                  90                  95


Asp Asp Val Asp Thr Lys Lys Gln Lys Thr Asp Glu Asp Asp
            100                 105                 110


<210> 58
<211> 105
<212> PRT
<213> Homo sapiens

<400> 58
Met Ser Asp Ala Ala Val Asp Thr Ser Ser Glu Ile Thr Thr Glu Asp
1               5                   10                  15

Leu Lys Glu Lys Lys Glu Val Val Glu Glu Ala Glu Asn Gly Arg Asp
            20                  25                  30

Ala Pro Ala His Gly Asn Ala Asn Glu Glu Asn Gly Glu Pro Glu Ala
        35                  40                  45

Asp Asn Glu Val Asp Glu Glu Glu Glu Glu Gly Gly Glu Glu Glu Gly
    50                  55                  60

Asp Gly Glu Glu Glu Asp Gly Asp Glu Asp Glu Gly Ala Glu Ser Ala
65                  70                  75                  80

Thr Gly Lys Arg Ala Ala Glu Asp Asp Glu Asp Asp Asp Val Asp Thr
                85                  90                  95

Gln Lys Gln Lys Thr Asp Glu Asp Asp
            100                 105

<210> 59
<211> 333
<212> DNA
<213> Homo sapiens

<400> 59
atgtcagacg cagccgtaga caccagctcc gaaatcacca ccaaggactt aaaggagaag      60

aaggaagttg tggaagaggc agaaaatgga agagacgccc ctgctaacgg gaatgctaat     120

gaggaaaatg gggagcagga ggctgacaat gaggtagacg aagaagagga agaaggtggg     180

gaggaagagg aggaggaaga agaaggtgat ggtgaggaag aggatggaga tgaagatgag     240

gaagctgagt cagctacggg caagcgggca gctgaagatg atgaggatga cgatgtcgat     300

accaagaagc agaagaccga cgaggatgac tag                                  333

<210> 60
<211> 318
<212> DNA
<213> Homo sapiens

<400> 60
atgtcagacg cagccgtaga caccagctcc gaaatcacca ccgaggactt aaaggagaag      60

aaggaagttg tggaagaggc ggaaaatgga agagacgccc ctgctcacgg gaatgctaat     120

gaggaaaatg gggagccgga ggctgacaac gaggtagatg aagaagagga agaaggtggg     180

gaggaagaag gtgatggtga ggaagaggat ggagatgaag atgagggagc tgagtcagct     240

acgggcaagc gggcagctga agatgatgag gatgacgatg tcgataccca gaagcagaag     300

accgacgagg atgactag                                                   318

<210> 61
<211> 18
<212> PRT
<213> Homo sapiens

<400> 61
Asp Ala Pro Ala Asp Glu Glu Asn Gly Glu Gln Glu Ala Asp Asn Glu
1               5                   10                  15

Val Asp

<210> 62
<211> 10
<212> PRT
<213> Homo sapiens

<400> 62
Asp Ala Pro Ala Asn Gly Asn Ala Val Arg
1               5                   10

<210> 63
<211> 5
<212> PRT
<213> Homo sapiens

<400> 63
Leu Ser Gln Leu Arg
1               5

<210> 64
<211> 5
<212> PRT
<213> Homo sapiens

<400> 64
Ala Ser Gly Gln Leu
1               5

<210> 65
<211> 7
<212> PRT
<213> Homo sapiens

<400> 65
Met Thr Met Ser Ile Pro Arg
1               5

<210> 66
<211> 4
<212> PRT
<213> Homo sapiens

<400> 66
Lys Lys Gln Lys
1

<210> 67
<211> 8
<212> PRT
<213> Artificial Sequence

<220>
<223> Description of Artificial Sequence: Synthetic
      His tag


<220>
<221> misc_feature
<222> (1)..(8)
<223> This sequence may encompass 6-8 "His" residues

<400> 67
His His His His His His His His
1               5


<210> 68
<211> 8
<212> PRT
<213> Artificial Sequence

<220>
<223> Description of Artificial Sequence: Synthetic
      His tag


<220>
<221> misc_feature
<222> (1)..(8)
<223> This sequence may encompass 4-8 "His" residues

<400> 68
His His His His His His His His
1               5


<210> 69
<211> 19
<212> PRT
<213> Homo sapiens

<400> 69
Glu Glu Glu Gly Asp Gly Glu Glu Glu Asp Gly Asp Glu Asp Glu Gly
1               5                   10                  15


Ala Glu Ser



<210> 70
<211> 6
<212> PRT
<213> Artificial Sequence

<220>
<223> Description of Artificial Sequence: Synthetic

6xHis tag

<400> 70
His His His His His His
1               5


<210> 71
<211> 10
<212> PRT
<213> Homo sapiens

<400> 71
Thr Lys Lys Gln Lys Thr Asp Glu Asp Asp
1               5                   10